(19) Europäisches Patentamt
European Patent Office
Office européen des brevets

(11) EP 1 387 699 B1

(12) **EUROPEAN PATENT SPECIFICATION**

(45) Date of publication and mention of the grant of the patent:
**29.11.2006 Bulletin 2006/48**

(21) Application number: **02742998.4**

(22) Date of filing: **13.05.2002**

(51) Int Cl.:
***A61K 47/34*** *(2006.01)* ***A61K 47/10*** *(2006.01)*

(86) International application number:
**PCT/EP2002/005242**

(87) International publication number:
**WO 2002/092130 (21.11.2002 Gazette 2002/47)**

(54) **AGENT FOR IMPROVING TISSUE PENETRATION**

MITTEL ZUR VERBESSERUNG DER GEWEBEPENETRATION

AGENT PERMETTANT D'AMELIORER LA PENETRATION TISSULAIRE

(84) Designated Contracting States:
**AT BE CH CY DE DK ES FI FR GB GR IE IT LI LU MC NL PT SE TR**

(30) Priority: **11.05.2001 DE 10122855**

(43) Date of publication of application:
**11.02.2004 Bulletin 2004/07**

(73) Proprietors:
- **MAX-PLANCK-GESELLSCHAFT ZUR FÖRDERUNG DER WISSENSCHAFTEN E.V.
80539 München (DE)**
- **GENZYME CORPORATION
Cambridge, MA 02142 (US)**

(72) Inventors:
- **EIBL, Hansjörg
37120 Bovenden-Eddigehausen (DE)**
- **HOFFMANN, Peter
Belmont, MA 02478 (US)**

(74) Representative: **Dey, Michael et al
Weickmann & Weickmann,
Patentanwälte,
Postfach 86 08 20
81635 München (DE)**

(56) References cited:
**WO-A-97/30058** **DE-A- 19 959 000**

- **PATENT ABSTRACTS OF JAPAN vol. 011, no. 038 (C-401), 4 February 1987 (1987-02-04) & JP 61 205277 A (POLA CHEM IND INC), 11 September 1986 (1986-09-11)**
- **PATENT ABSTRACTS OF JAPAN vol. 009, no. 145 (C-287), 20 June 1985 (1985-06-20) & JP 60 028946 A (KAO SEKKEN KK), 14 February 1985 (1985-02-14)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 1 387 699 B1

## Description

The invention concerns a pharmaceutical preparation which improves penetration of the active substance through the tissue membrane or barrier of the target organ.

The challenge in developing new pharmaceuticals is identifying agents that are both pharmacologically active agents and can reach the target site in the subject being treated. "Reaching the target site" is not only limited to the drug contacting the desired organ, but also requires the drug to contact particular cells in the organ, e.g., cancer cells, or to contact a significant percentage of the organ's cells. To achieve this result, the drug must penetrate throughout the tissues in the organ. In many instances, it is also necessary that the pharmacologically active agent cross the cell membrane of these cells to reach its biological target.

A well-known problem when administering pharmaceutical preparations is that the actual active substance cannot readily pass through the cell membrane and consequently the potential effects of the pharmaceutical preparation cannot be achieved in practice or the active substance has to be overdosed to such an extent that it increases the undesired side effects especially in organs other than the target organ.

In this respect the so-called blood-brain barrier is particularly problematic. The normal blood-brain barrier is a highly selective permeability barrier which impedes the blood-brain transfer of many compounds. The ability of an active substance in the blood stream to penetrate the blood-brain barrier largely depends on the ability of the active substance to separate itself from the blood and penetrate into the lipid of the endothelial cell plasma membranes. If there is not a specific mechanism, lipid solubility is the essential factor which determines the penetration of the active substance through the blood-brain barrier. In addition, molecules such as proteins having a molecular weight greater than about 500 daltons generally are not able to penetrate the blood-brain barrier, even if they are readily soluble in lipids.

There are many diseases and conditions of the central nervous system, e.g., Alzheimer's Disease, cancer, genetic disorders, stroke, trauma and depression, for which present treatments are ineffective. In vitro assays using targets isolated from the brain have been used to identify drug candidates for the treatment of these disorders. However, many of these drug candidates have failed when tested clinically because of their inability to penetrate the blood brain barrier. One strategy for overcoming this problem is to coadminister these compounds with a second agent as part of a pharmaceutical composition that enhances uptake by the brain. Unfortunately, there are few known pharmaceutical composition which increase penetration of the blood brain barrier.

It has also already been proposed that drugs should be chemically modified by attaching a residue having a high lipid solubility which facilitates penetration into the barrier. If this group is selected appropriately it would be cleaved again by the metabolism to release the active substance in its active form.

A disadvantage of this concept is that it is necessary to modify the actual active substance which may be difficult to carry out and, in view of the fact that the efficacy of pharmaceutically active substances is sensitive to changes in the molecule, this may result in impairment of the efficacy or lead to new undesired side effects.

Difficulties like those described for the blood-brain barrier also apply to other organs such as the liver, skin etc.

An objective of the invention was therefore to solve this problem in a simple manner without changing the actual active substance.

It has now been found that specific compounds, such as alkyl or acyl polyglycerols can open the spaces between cells in biological membranes, including the cells of the blood brain barrier. For example, treatment of primary cultures of monolayers of porcine brain microvascular endothelial cells (PBEC), an in vitro model of the blood brain barrier, with alkyl polyglycerols such as hexyldiglycerol significantly increased the monolayers' permeability towards compounds such as glucose, inulin and glycerol (Example 4). Normally, these compounds do not cross the blood brain barrier. Based on these findings, novel pharmaceutical compositions and methods of delivering a pharmacologically active agent to a target site in a subject are disclosed herein.

One embodiment of the present invention is a pharmaceutical composition. The pharmaceutical composition according to the invention comprises a compound of the formula (I):

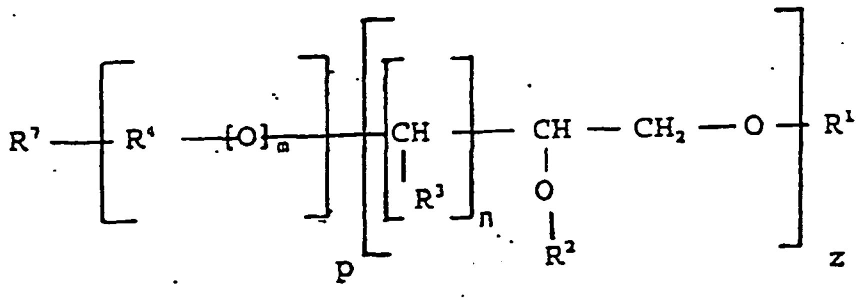

wherein:

$R^1$, $R^2$, $R^6$, $R^8$ and $R^9$ independently at each occurrence represent hydrogen or a linear or branched, saturated or unsaturated, substituted or unsubstituted hydrocarbon or acyl group, provided that at least one of $R^1$ and $R^2$ is H,
$R^3$ independently at each occurrence represents H, OH or $-O-R^9$,
$R^4$ independently at each occurrence represents $-(CH_2)_x-$ or $-CH_2[CH(R^5)-]_yCH_2-$,
$R^5$ independently at each occurrence represents H, OH, $R^6$ or $-O-R^6$,
$R^7$ represents H, OH, $CH_3$ or $-O-R^8$,
n is an integer from 0 to 6,
m is 0 or 1,
p is an integer from 1 to 9,
x is an integer from 0 to 12,
y is an integer from 1 to 4 and
z is an integer from 1 to 8.

The term "hydrocarbon group", as used herein preferably comprises alkyl, alkenyl and alkynyl groups, having 1 to 48 carbon atoms, in particular 1 to 24 carbon atoms. For some embodiments short chain hydrocarbon groups having 1 to 8 hydrocarbon atoms are preferred. In other embodiments long chain groups having 12 to 24 carbon atoms provide advantages.

The term "acyl group" refers to a hydrocarbon group, which has a -CO- group at its end.

Suitable substituents for the hydrocarbon or acyl group are e.g. alkoxy (in particular $C_1$-$C_8$ alkoxy), hydroxy or halogen, preferably $C_1$-$C_8$ alkoxy or hydroxy.

In one embodiment of the invention $R^3$ is preferably H. In another embodiment $R^3$ is preferably OH.
$R^5$ preferably represents OH.
$R^7$ preferably represents $R^8$-O-, wherein $R^8$ is a $C_1$-$C_{22}$-, in particular a $C_4$-$C_{11}$- alkyl or acyl group.
n can be an integer from 0 to 6 and is preferably an integer from 1 to 5, in particular from 1 to 4. For compounds of formula (I) having a group derived from a glycerol residue at its end n is preferably 1.

In one embodiment m is preferably 1. Particular preferred are such compounds having units being derived from ethylene oxide, propylene oxide and/or glycerol. In another embodiment m is preferably 0, including compounds having terminal alkane diols or alkane triols.
p is preferably an integer from 1 to 9, more preferably at least 2, more preferably at least 3 and up to 9.
x is an integer from 0 to 12, more preferably from 1 to 12, still more preferably from 3 to 12 and most preferably from 4 to 10.
y is an integer from 1 to 4 and most preferably 1.
z is an integer from 1 to 8, more preferably from 2 to 8 and most preferably from 3 to 8.

The symbols for the residues and numbers of residues used herein are independently at each occurrence within the formula, which means that within one formula a residue termed with the same symbol (e.g. $R^4$) can have a different meaning at each occurrence.

The pharmaceutical composition preferably comprises a compound represented by formula (II):

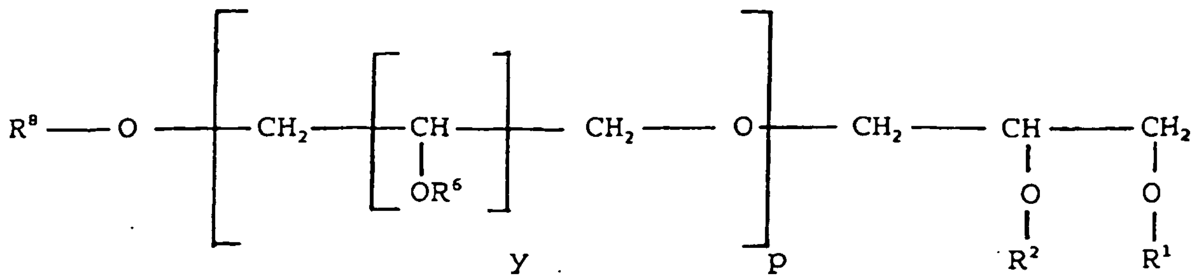

wherein:

$R^1$, $R^2$, $R^6$ and $R^8$ are defined as in claim 1,
y is an integer from 1 to 4; and
p is an integer from 1 to 9.

Particularly preferred are compounds in which n is 1, z is 1, m is 1, $R^3$ is H, $R^4$ is $-CH_2[CH(R^5)-]_yCH_2-$, $R^5$ is $-O-R^6$ and/or $R^7$ is $-O-R^8$.

These compounds have a unit derived from glycerol at one end and contain preferably at least one further glycerol unit. Particular preferred is hexyl diglycerol, e.g. 1-hexyldiglycerol or 2-hexyldiglycerol.

Particularly preferred are compounds having the formula (IIa)

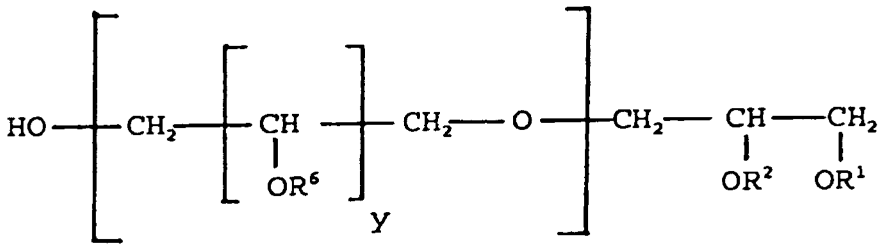

wherein:

$R^1$ and $R^2$ are independently a substituted or unsubstituted aliphatic group or -C(O)-(substituted or unsubstituted aliphatic group), provided that one of $R^1$ or $R^2$ is -H.
Each $R^6$ is -H or a substituted or unsubstituted aliphatic group or a substituted or unsubstituted acyl group and is independently selected.
y is an integer from 1 to 4.
p is an integer from 1 to 9.

In a further preferred embodiment of the invention the pharmaceutical composition comprises a compound of formula (III):

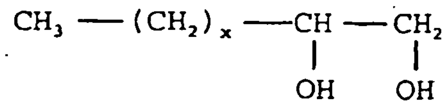

wherein:

x is an integer from 1 to 12, more preferably from 3 to 12. These compounds comprise a terminal alkane diol.

For many applications compounds in which $R^1$ is H, $R^2$ is H, n is 0, z is 1, p is 1, m is 0, $R^4$ is $-(CH_2)_x-$ or/and $R^7$ is $CH_3$ are preferred.

Further, pharmaceutical compositions are preferred comprising a compound of formula (IV):

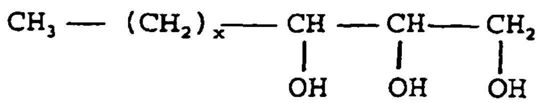

wherein:

x is an integer from 1 to 12, more preferably from 3 to 12.

These compounds contain terminal alkane triols.

Particular preferred are compounds wherein $R^1$ is H, $R^2$ is H, n is 1, $R^3$ is - OH, p is 1, m is 0, $R^4$ is $-(CH_2)_x-$ and/or $R^7$ is $-CH_3$.

In a further preferred embodiment the pharmaceutical composition of claim 1 comprises a compound of formula (V):

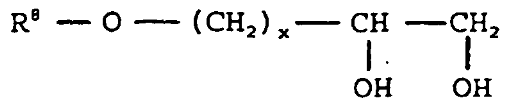

wherein:

$R^8$ is defined as above, and
x is an integer from 1 to 12. For compounds of formula (V) in one embodiment $R^8$ is preferably H. Then x is most preferably 6 to 13. In another embodiment $R^8$ for compounds of formula (V) is $C_2$-$C_{22}$-alkyl (resulting in an ether compound) or $C_2$-$C_{22}$-acyl (resulting in an ester compound) and then x is preferably 2 to 5.

Therefore, in further embodiments $R^1$ is preferably H, $R^2$ is preferably H, n is 0, z is 1, p is 1, m is O, $R^4$ is $-(CH_2)_x-$ and/or $R^7$ is $-O-R^8$.

In a further preferred embodiment the pharmaceutical composition comprises a compound of formula (VI):

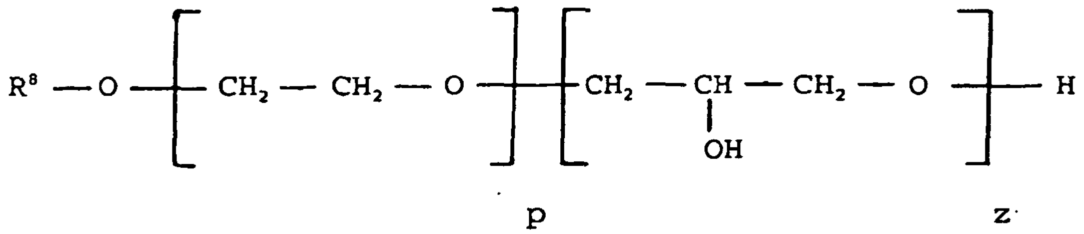

wherein:

$R^8$, p and z are defined as above. These compounds comprise ethylene glycol as well as glycerol units. In this embodiment p is preferably an integer from 1 to 4, z is preferably an integer from 1 to 3 and $R^8$ is preferably $C_1$-$C_{22}$, in particular $C_2$-$C_{12}$ alkyl or acyl.

Therefore, in further embodiments $R^1$ is preferably H, $R^2$ is H, n is 1, $R^3$ is H, m is 1, $R^4$ is $-(CH_2)_x-$, x is 2 or/and $R^7$ is $-O-R^8$.

Also combinations having first a glycerol unit and then an ethylene glycol unit are possible as well as mixed arrangements, such as e.g. $R^8$-O-ethylene glycol$(E)_1$-O-glycerol$(G)_1$-O-$(E)_2$-O-$(G)_2$.

In a further preferred embodiment the pharmaceutical composition comprises a compound of formula (VII):

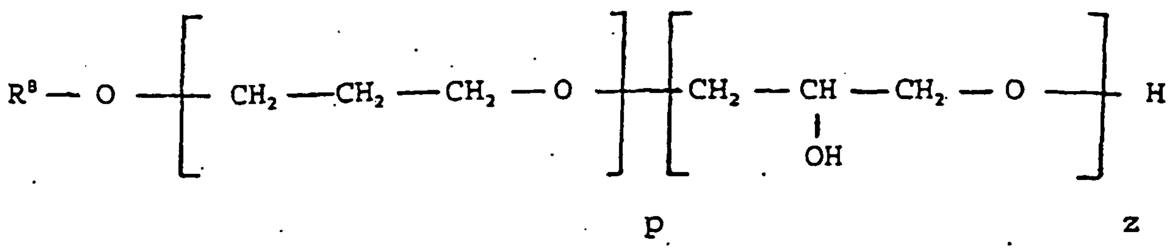

wherein:

$R^8$, p and z are defined as above. These compounds comprise polypropylene glycol (P) units in combination with glycerol (G) units. Preferably $R^8$ is $C_1$-$C_{22}$-, in particular $C_2$-$C_{12}$-alkyl or acyl, p is 1 to 4 and z is 1 to 3. Also combinations having first glycerol are possible.

Therefore, in a further embodiment $R^1$ is preferably H, $R^2$ is H, n is 1, $R^3$ is H, m is 1, $R^4$ is $-(CH_2)_x-$, x is 3 or/and $R^7$ is $-O-R^8$.

In a further preferred embodiment the pharmaceutical composition of the invention comprises a compound of formula (VIII):

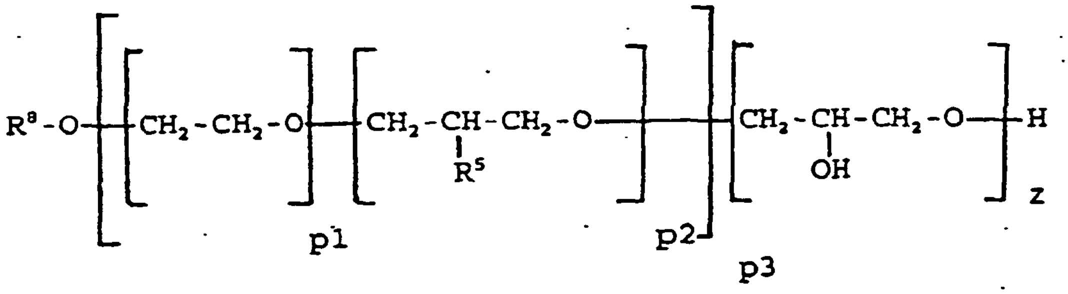

Wherein:

$R^8$, $R^5$ and z are defined as above and p1 is an integer from 0 to 20, p2 is an integer from 0 to 20 and p3 is an integer from 0 to 10, with the proviso that, p1 + p2 ≥ 1 and with the proviso that, if p1 is 0 at least one $R^5$ is H.

The compounds are particularly three-fold combinations with the units ethylene glycol, glycerin and propylene glycol. Such compounds allow particular a fine adjustment of the physical properties and an equal balance between lipophilic and hydrophobic regions of the molcules. $R^5$ is preferably H or OH.

Therefore, in further embodiments compounds are preferred wherein $R^1$ is H, $R^2$ is H, $R^3$ is H, n is 1, m is 1 and/or $R^7$ is $-O-R^8$.

In a further preferred embodiment the composition of the invention comprises a compound of formula (IX):

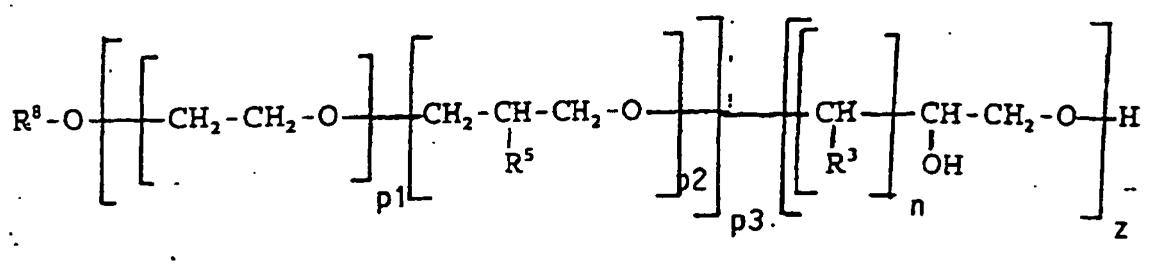

wherein:

$R^3$, $R^5$, $R^8$ and z are defined as above. p1 is an integer from O to 20, p2 is an integer from 0 to 20, p3 is an integer from 1 to 10 and n is an integer ≥ 2. These compounds are molecules containing a terminal sugar alcohol residue and as further units ethylene glycol, glycerin or/and propylene glycol. $R^3$ is preferably H or OH, $R^5$ is preferably H or OH. Preferably p1 + p2 ≥ 1 and, if p1 = 0 at least one $R^5$ = H.

Therefore, compounds are preferred in which $R^1$ is H, $R^2$ is H, m is 1 and/or $R^7$ is $-O-R^8$.

The present invention further relates to a pharmaceutical preparation which is composed of an active substance in combination with at least one compound of general formula (I) as described above. This composition may further comprise common pharmaceutical additives and/or diluents.

Another embodiment of the present invention is a method of delivering a pharmacologically active agent to a target site in a subject. Examples of target sites include the brain, the gastrointestinal tract, the skin, the lungs or liver. The method comprises administering an effective amount of the pharmaceutical composition described above.

Another embodiment of the present invention is a pharmaceutical composition, as described above, for use in therapy, for example, to treat disorders of the brain, gastrointestinal tract, skin, lungs or liver.

Yet another embodiment of the present invention is the use a compound represented by formula (I), preferably in combination with a pharmaceutically active agent for use of the manufacture of a medicament. The medicament can be used in therapy, for example, for the treatment of disorders of the brain, gastrointestinal tract, skin, lungs or liver.

The disclosed pharmaceutical compositions open the spaces between cells and allow compounds such as

drugs to penetrate into and through-out tissue and organs and even across cell membranes. As a consequence, the bioavailability of compounds to their target sites is increased. In particular, these pharmaceutical compositions facilitate uptake through the blood brain barrier of pharmacologically active compounds which otherwise would not enter brain, e.g., proteins, nucleic acids and hydrophilic small molecule drugs. They can therefore be used in conjunction with these pharmacologically active compounds to treat variety of disorders of the central nervous system, such as cancer, Alzheimer's Disease, genetic diseases, stroke, trauma and depression. In addition, the disclosed pharmaceutical composition can further enhance uptake of drugs currently being used to treat these disorders, thereby allowing their administration in lower doses. Uptake of pharmacologically active agents into other organs such as the skin, lungs, liver and intestines is also facilitated by the disclosed pharmaceutical compositions.

The disclosed pharmaceutical compositions enhance uptake of biologically active agents into the brain and other organs. These pharmaceutical compositions preferably comprise a biologically active agent and a compound referred to herein as an "uptake enhancer". The uptake enhancer is represented by formula (I).

In a preferred embodiment, the variables in formula (I) are defined as follows:

$R^1$ and $R^2$ are independently H or a $C_1$-$C_{22}$ alkyl, alkenyl, alkynyl or acyl group, provided that one of $R^1$ or $R^2$ is -H; each $R^6$ is -H or a $C_1$-$C_{22}$ alkyl, alkenyl, alkynyl or acyl group and is independently selected; and p is an integer from 1 to 6. More preferably, $R^6$ is -H.

In a more preferred embodiment, the uptake enhancer is represented by formula (X):

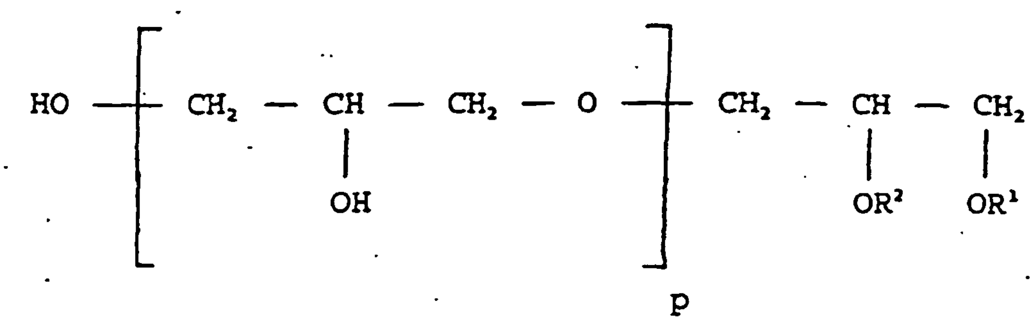

In formula (X), $R^1$, $R^2$ and p are as described above. Preferably, $R^1$ is $C_4$-$C_{12}$ alkyl and $R^2$ is -H. p is preferably 2 or 3.

Specific examples of uptake enhancers include 3-(3-hexyloxy-2-hydroxy-propoxy)-propane-1,2-diol or 3-[2-hydroxy-3-(2-hydroxy-2-octyloxy-propoxy)-propoxy]-propane-1,2-diol.

In a preferred embodiment the invention relates to a pharmaceutical preparation which is characterized in that it is composed of an active substance in combination with at least one compound of the general formula (XI):

$H_2C$ - O - $R_2$

$H_2C$ - O - $R_2$

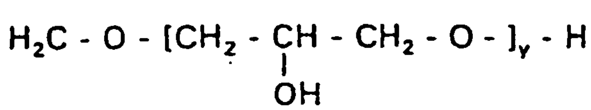

in which one of the residues $R_1$ and $R_2$ denotes an alkyl, alkenyl, alkinyl or alkoyl group each having 1 to 22 C-atoms and the other residue denotes a H atom, and common pharmaceutical additives and diluents.

The compound of the general formula is a glycerol derivative which is substituted in position 1 or in position 2 with one of the above-mentioned short-chain groups. The substituents can be straight-chained or branched and optionally also be cyclic and contain up to two double or triple bonds.

The symbol z in the general formula (XI) denotes a number from 1 to 6 and in particular 2 or 3.

Preferred applications of the compounds are stated in the claims. Preferred are pharmaceutical preparations in which $R^1$ or $R^2$ has 7 to 22 C atoms when the active substance is not surface-active or pharmaceutical preparations

in which $R^1$ or $R^3$ has 1 to 6 C atoms when the active substance is surface-active.

The oligoglycerol derivatives according to the invention surprisingly exhibit an improved effect compared to the monoglycerol derivatives known from EP 0 144 069 especially with regard to the fine adjustment of lipophilic/hydrophilic properties.

The compounds of the invention enhance delivery through biological membranes. This effect can be observed in PBEC (porcine brain endothelial cells) cell culture, where certain concentration will open up the spaces between cells in order to allow compounds like various drug to penetrate into and through-out tissue and organs. Some of those called junction are very tight especially what constitutes part of the blood brain barrier.. It could even been shown that the blood brain barrier, which is one of the most difficult membrane to penetrate can be overcome. Thereby drug could be made bioavailable for treatment of various diseases that affect the brain tissue. Most compounds - drugs that have been tried will enter into the brain, if mixed together with any of the compounds of the invention. On a cell based assay it could be demonstrated that the tight junction - spaces between the cells become wider - opening up and various drugs that otherwise can not enter into the hemisphere of the brain can enter into the brain. The drugs will then distribute throughout the brain and can exhibit their corrective action as designed.

The compounds of the invention are in particular able to penetrate brain, liver, spleen, kidney, heart, intestine, lung and eyes. Preferably, they are applied to penetrate blood-brain barrier or blood-occutar barrier. The compositions of the invention can be used in gene therapy using plasmids, vectors or oligonucleotides, in antisense therapy using oligonucleotides or peptide-nucleotide as well as in cell therapy using fragments or whole cells.

The term "aliphatic group" as used herein comprises a straight chained or branched hydrocarbon which is completely saturated or which contains one or more units of unsaturation. Typically, a straight chained or branched aliphatic group has from 1 to about 22 carbon atoms and preferably from 1 to about 10. An aliphatic group is preferably a straight chained or branched alkyl group, e.g, methyl, ethyl, n-propyl, n-butyl, n-pentyl, n-hexyl, n-heptyl or n-octyl.

An alkenyl group is a straight chain or branched aliphatic group having one or more double bonds, preferably one double bond; and an alkynyl group is an aliphatic group with one or more triple bonds, preferably one triple bond.

An acyl group (substituted or unsubstituted) is represented by -C(O)-R, wherein R is a substituted or unsubstituted aliphatic group. An acyl group is also referred to as an "alkanoyl group".

Suitable substituents for an aliphatic groups are those which do not substantially interfere with the ability of the uptake enhancer to promote uptake of pharmacologically active agents by a target organ, preferably the brain, e.g., decrease uptake by more than 50% compared with the corresponding uptake enhancer which does not have the substitutent. Examples of suitable substituents include C1-C3 alkyl groups, halogens, $C_1$-$C_3$ alkoxy groups and hydroxy groups.

A "target site" is a site within the body of a subject which is in need of treatment with a pharmacologically active agent, i.e., a drug. A target site for example can be an organ, specific tissue within the organ and/or specific cells within the organ. The methods disclosed herein can facilitate uptake of pharmacologically active agents by specific organs and permeation of the agents throughout said organs, resulting in the delivery of the agent to specifically targeted tissue and cells.

A wide variety of pharmacologically active agents are suitable for use in the pharmaceutical compositions of the present invention. Such agents include protein drugs, nucleic acid drugs and small molecule drugs.

When the pharmaceutical compositions of the present invention are used to facilitate uptake into the brain, pharmacologically active agents currently used to treat disorders of the brain, as well compounds which normally cannot pass through the blood brain barrier, are generally suitable. Thus, the disclosed pharmaceutical compositions can further enhance the effectiveness and/or lower the amount which is therapeutically effective for drugs currently used. The compounds of the invention can particularly be used for the preparation of pharmaceutical compositions, optionally in combination with an active substance, for the treatment of CNS trauma; hemorraghic trauma; infection/antibiotics; meningitis, aseptic; meningitis, bacterial; meningitis, cryptococcal; meningitis, meningococcal; stroke/traumatic brain injury; brain cancer; brain/nerve disorder (misc); cerebrovascular accident (CVA); dementia; encephalitis; anti-bacterial; antiviral; anxiety; attention deficit syndrome; auto-immune disease (non-specific); bipolar disorder; brain cancer; brain/nerve disorder (misc); cerebrovascular accident (CVA); CNS trauma; cytomegalovirus (CMV); dementia; depression; encephalitis; epilepsy; Fabry's disease; fungal infection (non-specific); Gaucher's disease; genetic disorder (misc); hemorraghic trauma; herpes simplex virus; HIV/AIDS; hormonal disorder (misc); inflammation (general); insomnia; lyme disease; meningitis, aseptic; meningitis, bacterial; meningitis, cryptococcal; meningitis, meningococal; mental health (misc); migraine; multiple sclerosis (MS); neoplastic diseases; pain control; panic disorder; Parkinson's disease; psychosis; schizophrenia; spinal cord injury; stroke/traumatic brain injury; tinea. Preferably, they are used to treat Alzheimer's Disease, cancers of the brain, genetic diseases, stroke, brain trauma and depression. Compounds which are active in vitro against targets isolated from the brain but which cannot cross the blood brain barrier are ideal candidates for use in the disclosed pharmaceutical compositions, including hydrophilic agents, compounds having a molecular weight greater than about 500 daltons, preferably active substances having a molecular weight in the range > 1500 Da, protein drugs and nucleic acid drugs. In addition, drugs which cannot cross the blood brain barrier but are used to treat disorders in other parts of

the body can enable treatment of similar disorders in the brain. For example, the anti-neoplastic drugs 5-fluorouracil, mitoxanthrone, etoposide, methotrexate, vinblastin, peplomycin or daunomycin, which do not cross the blood barrier, have increased availability to the brain when administered as part of the disclosed pharmaceutical compositions.

As discussed previously, the disclosed pharmaceutical compositions can also be used to target organs other than the brain. Successful delivery to a selected target can be improved by the mode of administration, as discussed below in greater detail. Examples of other organs which can be targeted include the lungs, intestines, skin and liver. As with the brain, the disclosed pharmaceutical compositions can increase the uptake and effectiveness of drugs currently used to treat diseases of these organs; can enable these organs to be treated with drugs that are currently used to treat disorders in other parts of the body but which are poorly bioavailable in these organs; and can enable treatment with compounds that are otherwise poorly absorbed by these organs but which are found to be active in vitro against targets isolated from these organs.

The disclosed pharmaceutical compositions can be administered to a subject by any means suitable for delivering the pharmacologically active agent to the target organ. For example, when the target organ is the brain, the pharmaceutical composition is delivered in a manner which allows the composition to enter the blood stream for delivery to the brain. Thus, intravenous or intraarterial administration is preferred, such as direct administration into the carotid artery. Sustained delivery pumps, as are well known in the art, can be advantageously used to administer the compositions to the carotid artery or other blood vessels. If a formulation containing a compound of the invention and a pharmaceutical is injected in close proximity to the blood-brain-barrier a large portion of the drug is delivered and distributed to one or both hemispheres, depending on the injection site. However, if the drug is administered in locations distant from the brain the compound of the invention still may facilitate to deliver small quantities into the CNS and by distribution make a drug including large bio-molecules bio-available.

Others modes of administration which deliver the disclosed pharmaceutical compositions to the target organ are also contemplated. Thus, parenteral, pulmonary, transdermal, ocular, oral and rectal administration can also be used. When released in the intestines, the disclosed pharmaceutical compositions can penetrate the intestinal membrane and make the pharmacologically active agent bioavailable in adjacent tissue or even systemically. Delivery to the intestines can be achieved by oral administration, provided that the composition is suitably coated for to pass through the stomach and be released in the intestines, and by rectal administration. Thus, oral and rectal administration can be used to target the intestines and brain. Similarly, the ability of the drug to penetrate the aviolae or lung tissue and allow the drug to enter the blood stream is enhanced by the disclosed pharmaceutical compositions when administered by pulmonary means. Thus, the pharmaceutical compositions of the present invention can be used to target the lungs and brain when administered by pulmonary means. Topical administration is used to target the skin.

When administered by pulmonary application, the disclosed pharmaceutical compositions can be delivered as a liquid formulation, dry powder or particle formulation. The formulation can be delivered, for example, in aerosolized form. Delivery of aerosolized therapeutics is known in the art (see, for example US Patent No. 5,767,068 to VanDevanter et al., U.S. Patent No. 5,508,269 to Smith et al., and WO 98/43650 by Montgomery, the entire teachings of which are incorporated herein by reference). Pharmaceutical compositions of the invention to be delivered as aerosols for pulmonary delivery are formulated such that an effective dose may be aerosolized (e.g. using a jet or ultrasonic nebulizer) to a particle size optimal for the desired treatment. Examples of a suitable particle size for delivery into the endobronchial space is generally about 1 to 5 microns.

As discussed above, when targeting the intestines by oral administration, the disclosed pharmaceutical compositions are preferably encapsulated with a coating to allow passage through the stomach. Suitable coatings are well known in the art and include hard gelatin or cyclodextran. These and other suitable encapsulation techniques are described, for example, in Baker, et al., "Controlled Release of Biological Active Agents", John Wiley and Sons, 1986, the entire teachings of are incorporated herein by reference. Optionally, other carriers or diluents commonly found in pharmaceutical formulations can be added to the disclosed pharmaceutical compositions, provided that uptake into the target organ and activity of the pharmacologically active agent is not adversely effected. Examples of suitable pharmaceutical carriers for parenteral administration include, for example, sterile water, physiological saline, bacteriostatic saline (saline containing about 0.9% mg/ml benzyl alcohol), phosphate-buffered saline, Hank's solution, Ringer's-lactate and the like and are described in Remington's Pharmaceutical Sciences, Mack Publishing Company, Easton, PA., the entire teachings of which are incorporated herein by reference.

For parenteral application the pharmaceutical compositions can be formulated, e.g. as liposomes, emulsions, mycels, complexes, suspensions (e.g. with particles, solid nanoparticles or solutions). For inhalation preferably dry powders, particles, solid nanoparticles, liposomes, emulsions, mycels, complexes, suspensions or solutions are used. For oral application capsules, tablets with interic coating containing e.g. dry powder, particles, solid nanoparticles, liposomes, emulsions, mycels, complexes, suspensions, self-emulsifying formulations or time-release formulations can be applied.

An "effective amount of the disclosed pharmaceutical composition" is the quantity which delivers a sufficient amount of the uptake enhancer to enable uptake of the pharmacologically active agent into the target organ (i.e., an

"effective amount of the uptake enhancer") and a sufficient amount of the pharmacologically active agent to have a beneficial therapeutic or prophylactic effect (i.e., an "effective amount of the pharmacologically active agent"). The precise amount of each typically depends on the target site, mode of delivery, on the pharmacologically active agent being used, the disorder being treated and the overall health, age and sex of the subject being treated, and can readily be determined by the skilled practitioner. Typically, between about 0.01 mg per kg per day and about 10 mg per kg per day of the pharmaceutical is administered to the subject, preferably between about 0.1 mg per kg and about 1 mg per kg.

The pharmaceutical compositions of the present invention can be prepared by mixing the uptake enhancer and the pharmacologically active agent. Generally, between about 1:100 w/w and 100:1 w/w of uptake enhancer to pharmacologically active agent are used, preferably between about 10:1 w/w and 1:10 w/w.

A "subject" is a mammal, preferably a human, but can also be an animal in need of veterinary treatment, e.g., companion animals (e.g., dogs, cats, and the like), farm animals (e.g., cows, sheep, pigs, horses, and the like) and laboratory animals (e.g., rats, mice, guinea pigs, and the like).

The preparation of the uptake enhancers used in the pharmaceutical compositions of the present invention is shown schematically is Figure 1. Isopropylidene glycerol is reacted with allyl glycidyl ether in the presence of a catalytic amount of sodium hydroxide to form Intermediate 1. The free secondary alcohol is then alkylated or protected, as appropriate, to form Intermediate 2. The double bond is then epoxidized with, for example, meta-chloroperbenzoic acid, to form Intermediate 3. If another glycerol unit is to be added, the epoxide is opened with allyl alcohol to form Intermediate 4, which can then undergo another cycle of epoxidation, protection and epoxide opening to add another glycerol unit. If no further glycerol units are to be added, the epoxide is preferably opened with benzyl alcohol, which can be cleaved by hydrogenation. The protecting groups can be removed at the end of the synthesis to form an uptake enhancer, as disclosed herein. Specific conditions for these reactions are provided in Examples 1-3.

The invention is further elucidated by the following figures and examples: Figure 1 (1 A and 1 B) is a schematic showing the synthesis of the uptake enhancers described herein.

EXEMPLIFICATION

Example 1

Preparation of 1,2-Isopropylidene-G1- 3,1-G2-O-Allyl Ether

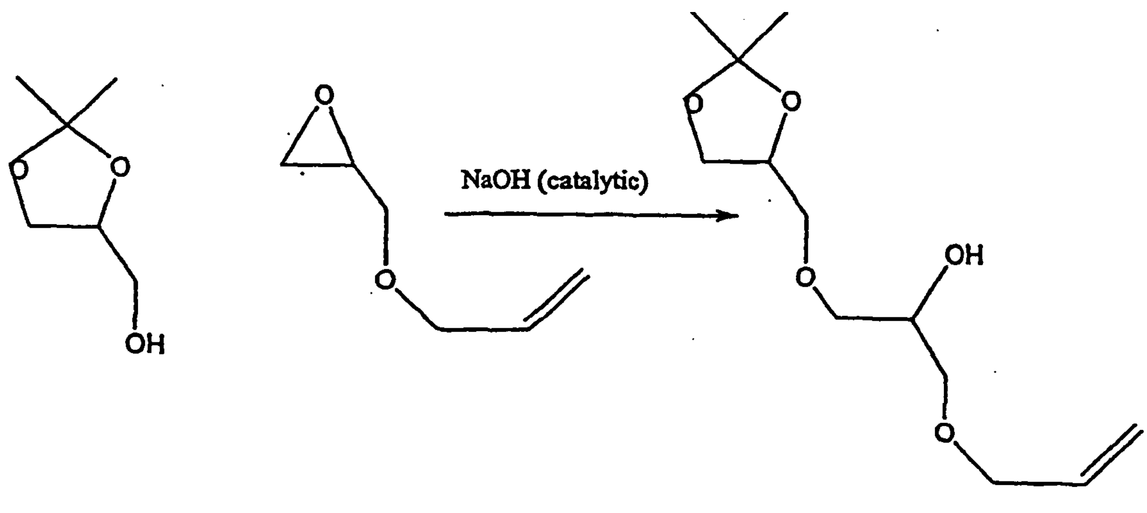


A catalytic quantity of NaOH (MW 40.00; 0.6 mol - 24 g) was added to 1,2-isopropylidene-rac-glycerol (MW 132.16; 16 mol - 2115 g) and dissolved by stirring and heating to 80° C. At 80° C , allyl glycidyl ether (MW 114.14; 6 mol - 685 g) was added dropwise over a period of two hours. The reaction mixture was stirred for another two hours at 80° C, at which point the epoxide (Rf in ether = 0.8) had reacted completely to form the G2 constituent (Rf in ether = 0.6). The excess isopropylidene-rac-glycerol had an Rf of 0.65 in ether and was removed from the reaction mixture at 75° C/10 mbar. About 1 liter of diisopropyl ether was added to the residue and the resulting solution was extracted twice with 1 liter NaCl (1% solution in H2O). The organic phase was removed in vacuo and the residue distilled (Kpi10-1 mbar 125° C).

The yield of the pure product 1,2-isopropylidene-rac-G1-3.10.0-3-0-allyl-rac-G2 (MW 246.30) was 1025 g (ca. 70%).

Instead of 1,2-isopropylidene-rac-glycerol, it is possible to react other primary alcohols and also allyl alcohol

and benzyl alcohol under the conditions described above. In the same manner, it is also possible to use other epoxides.

Example 2

Preparation of 1,2-Isopropylidene-G1- 3,1-G2-2-O-Benzyl-O-Allyl Ether

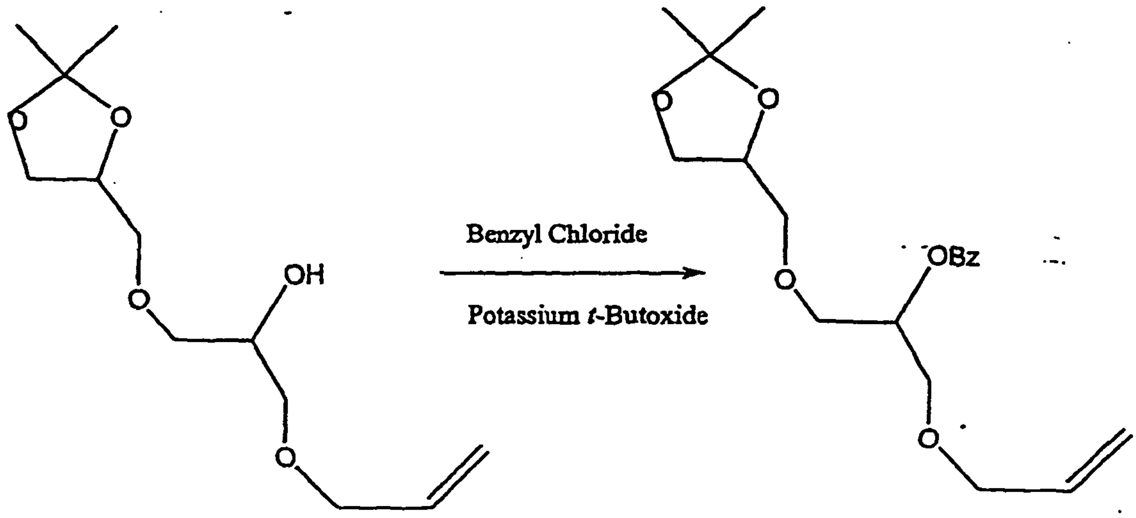


1,2-isopropylidene-rac-G1-3.1-rac-G2-0-allyl ether (MW 246.30; 0.5 mol - 123 g), obtained from Example 1, and benzyl chloride (0.6 mol - 76 g) were dissolved in 500 ml tetrahydrofuran and refluxed. Potassium tert-butoxide(0.7 mol -79 g) dissolved in 500 ml tetrahydrofuran was added dropwise to the reaction mixture. After thrity minutes of reflux, the reaction was completed. One liter of diisopropyl ether and 1 liter of 1 % NaCl solution was added to the reaction mixture. The mixture was shaken, the organic layer was separated and the solvent removed in a rotary evaporator. The product can either be used directly, or recovered in pure form in approximately 90% yield by means of chromatography on silica gel. Empirical formula: C19H28O5 (MW 336.42). Calculated: C, 67.83; H, 8.39; O, 23.79; measured: C, 67.78; H, 8.34; O.

Instead of benzyl chloride, use can also be made of benzyl bromide, allyl chloride or allyl bromide, or of the mesylates of primary alcohols. The products of the reaction between primary or secondary hydroxyl groups and alkyl mesylates, in particular, lead to high yields (>90%) of the desired target compounds.

Example 3

Epoxidation of 1,2-Isopropylidene-G1- 2-O-Benzyl-3,1-G2-O-Allyl Ether

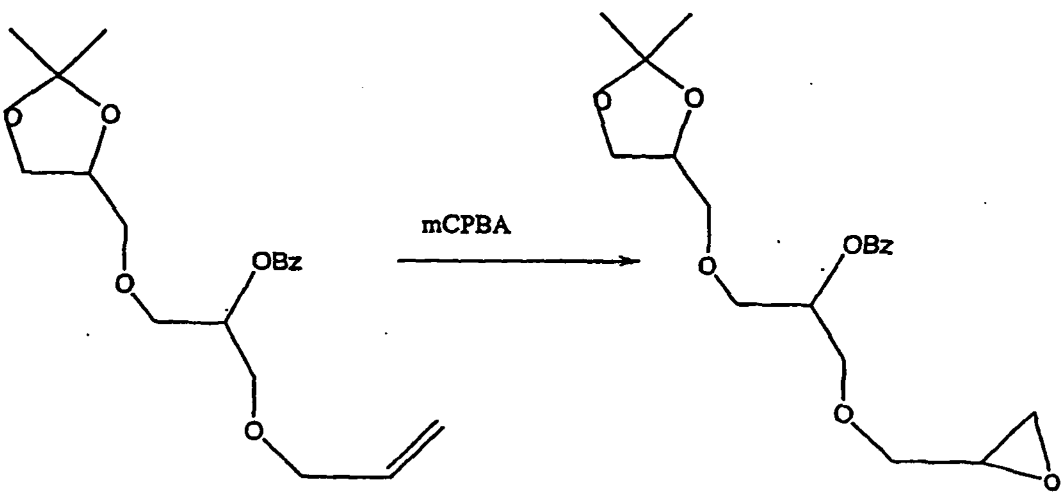


1,2-isopropylidene-rac-glycero-2-O-benzyl-3-0-allyl ether (1 mol) was dissolved in 1 liter CH2Cl2. 3-Chloroperoxybenzoic

acid (1.1. mol) was added portion-wise and the reaction mixture was stirred for six hours at 25 - 30° C. The starting material (Rf 0.5 in diethyl ether/pentane 1:1) was by then transformed completely into the desired product (Rf 0.2 in the above system). After removing the precipitate by suction filtration, 100 g Na2C03 was added to the filtrate and the mixture stirred for another three hours at 20° C. The precipitate was removed and the solvent removed under vacuum. The yield of epoxide (MW 188.22) was 170 g (90%).

Example 4

Hexyl Diglycerol Increases Permeability of the Blood Brain Barrier in an In Vitro Model

Monolayers of primary cultures of porcine brain microvascular endothelial cells (PBEC) represent an in vitro model of the blood-brain barrier. They form monolayers under standard culture conditions both on various collagen coated solid substrates and permeable filters of polycarbonate. The PBEC-monolayer grows on the filter membranes in polarized manner with the apical side representing the capillary lumen but the basolateral side corresponding to brain tissue. As soon as the cells become confluent and build up a tight layer after 7 days PBEC are ready to be used for transport experiments. Due to the formation of functional tight junctions, they show a high transendothelial electrical resistance (TEER) and a tight barrier is created with biological properties similar to the cerebral capillary endothelium

The PBEC cells were cultivated in M 199, which was supplemented with 10% OS, 0.7 mM $L^{-1}$ glutamine, 10,000 $U{\cdot}mL^{-1}$ penicillin/streptomycin and 50-$\mu$g $mL^{-1}$ gentamicin at 37° C, 5% $CO_2$ and saturated humidity. The cells were subcultivated after being detached with trypsin-EDTA solution and sown on Transwell(r) filter inserts (Costar®, Wiesbaden, Germany). The filters consist of polycarbonate with a surface of 1.13 cm2 and a pore diameter of 0.4 $\mu$m.

The growth of the PBEC into confluent, differentiated monolayers on Transwell® filter inserts was verified by measurements of the TEER. After 7 days, the integrity of the monolayers was confirmed by the transport of two marker substances: fluorescein and propranolol. Fluorescein passes the monolayer paracellularly, whereas propranolol is transported transcellularly. The transport studies were carried out directly on the Transwell® plates. The culture medium M199 in the apical and the basolateral compartment was replaced by serum free medium DME/Ham's F12 supplemented as described for M199 and with 0.2 $\mu g{\cdot}mL^{-1}$ hydrocortisone one day prior to the experiment. All transport experiments involving cell-cultures were carried out as triplicates. For application of substances on the apical side half of the medium of aspirated, mixed with appropriate stock solution of the substance to be investigated and replaced onto the filter. For qualification of transcellular transport by propranolol, KRB replaced the DME/Ham's F12 as transport buffer. For transport across cell free filter inserts, the complete medium in the apical compartment was removed, mixed with stock solution and replaced into the donor compartment. Stock solutions of inuline and FITC-dextran were prepared in $H_2O$. Sucrose was delivered as 3% ethanolic solution, glycerol as 50% ethanolic solution. Propranolol was dissolved in KRB (pH 7.4). Samples were collected at 15', 30', 45', 60', and 90' from the acceptor, at 0' and 90' from the donor compartment and the sample volume was replaced by fresh transport buffer. Prior to the experiment and after the final sampling, the TEER of the monolayers was measured.

The apparent permeability coefficient ($P_{app}$, [$cm{\cdot}s^{-1}$]) was calculated according to equation 1, where dQ/dt is the permeability rate (steady state transport rate, [$\mu g{\cdot}s^{-1}$]) obtained from the profile of the transported amount of the substrate against the time. A (1.13 $cm^2$) is the surface of the exposed cell monolayer, m0 the original mass [$\mu$g] of the marker substance in the donor compartment ($V_{Donor}$ 0.5 mL). The effective barrier function of the cell-layer is calculated from the apparent permeation coefficient and the permeation coefficient of the cell-free filter according to equation 2. In case that $P_{app}$ is of higher magnitude than $P_{filter}$, negative permeability coefficients would be calculated. Negative values for $P_{eff}$ are unsensical, thus an unhindered penetration of the cell-layer is to be drawn as conclusion.

equation 1
$$P_{app} = \frac{dQ}{dt} \cdot \frac{1}{m_0} \cdot \frac{1}{A} \cdot V_{donor}$$

equation 2
$$\frac{1}{P_{eff}} = \frac{1}{P_{app}} - \frac{1}{P_{filter}}$$

In order to find an appropriate concentration affecting the permeation across the BBB, four different concentrations of hexyldiglycerol (HexylG2) were checked for their influence on fluorescein permeation and transendothelial electrical resistance. 0.1 mM, 1 mM and 10 mM of HexylG2 turned out to have no effect on the tightness of the model barrier. Application of 50 mM enhanced the penetration of fluorescein about twofold, indicating a permeation enhancing effect at this concentration. Permeation data given as $P_{eff}$ and $P_{app}$ are summarized in Table 1. Since a more distinct effect was expected from in vivo findings, a concentration of 75 mM HexylG2 was selected to be tested with further impermeable markers.

Table 1: Permeability coefficients for fluorescein at different concentrations of HexylG2.

| conc. HexylG2 | $P_{eff}$ [cm- s$^{-1}$] | $P_{app}$ [cm·s$^{-1}$] | RSD | n = |
|---|---|---|---|---|
| w/o | $2.9\cdot10^{-7}$ | $2.8\cdot10^{-7}$ | 43% | 3 |
| 100 μm | $3.0\cdot10^{-7}$ | $2.9\cdot10^{-7}$ | 14% | 3 |
| 1 mM | $2.2\cdot10^{-7}$ | $2.2\cdot10^{-7}$ | 21% | 3 |
| 10 mM | $3.9\cdot10^{-7}$ | $3.8\cdot10^{-7}$ | 39% | 3 |
| 50 mM | $6.5\cdot10^{-7}$ | $6.2\cdot10^{-7}$ | 9% | 3 |

As can be seen from the data in Table 1, a twofold elevation of fluorescein permeability accompanied by a decrease in TEER indicates that HexylG2 can open the blood brain barrier.

Sucrose, glycerol, FITC-dextran (4 kDa), and inulin were tested for their ability to penetrate the PBEC monolayer in the presence and absence of HexylG2. Normally, these compounds poorly penetrate the BBB. It was found that application of HexylG2 leads to a drastic increase of permeation velocity: $P_{app}$-values for all compounds, when tested in combination with HexylG2 in the range of 1 - $5\cdot10^{-5}$ cm·s$^{-1}$, thus being very similar to the permeability of propranolol, a substance that crosses the BBB unhindered. In addition a complete loss of TEER was also observed. This clearly indicated an unspecific opening of the barrier. Permeation data given as $P_{eff}$ and $P_{app}$ are summarized in Table 2.

Table 2: Permeability coefficients observed with and w/o HexylG2 for paracellular-transport markers. (n.s. = no signal; n.c. = could not be calculated since $P_{app} > P_{eff}$)

| substance | $c_0$[μg·mL$^{-1}$] | $P_{eff}$[cm-s$^{-1}$] | $P_{app}$[cm-s$^{-1}$] | RSD | n= |
|---|---|---|---|---|---|
| w/o HexylG2 | | | | | |
| $^{14}C$-sucrose | | $1.1\cdot10^{-6}$ | $1.1\cdot10^{-6}$ | 12% | 3 |
| $^{14}C$-glycerol | | $9.6\cdot10^{-7}$ | $8.1\cdot10^{-7}$ | 41% | 3 |
| $^{3}H$-inulin | | $2.4\cdot10^{-7}$ | $2.4\cdot10^{-7}$ | 16% | 3 |
| FITC-dextran 4kDa | | n.s. | n.s. | - | 3 |
| 75 mM HexylG2 | | | | | |
| $^{14}C$-sucrose | | $2.2\cdot10^{-4}$ | $4.4\cdot10^{-5}$ | 9% | 3 |
| $^{14}C$-glycerose | | n.c. | $4.4\cdot10^{-5}$ | 7% | 3 |
| $^{3}H$-inulin | | $7.7\cdot10^{-5}$ | $3,7\cdot10^{-5}$ | 8% | 3 |
| FITC-dextran 4 kDa | | $4.1\text{-}10^{-5}$ | $1.7\text{-}10^{-6}$ | 14% | 3 |

Example 5:

Group A

Pentanediol- (1.2)

Hexanediol- (1.2)
Heptanediol- (1.2)
Octanediol- (1.2)
Nonanediol- (1.2)
Decanediol- (1.2)
Hexadecanediol- (1.2)
Octadecanediol- (1.2)
Eicosanediol- (1.2)

Group B

Pentanetriol- (1.2.3)
Hexanetriol- (1.2.3)
Hepanetriol- (1.2.3)
Octanetriol- (1.2.3)
Nonanetriol- (1.2.3)
Decanetriol- (1.2.3)

Group C

$R^8$ = H

Butanetriol- (1.2.4)
Pentanetriol- (1.2.5)
Hexanetriol- (1.2.6)
Heptanetriol- (1.2.7)
Octanetriol- (1.2.8)
Nonanetriol- (1.2.9)
Decanetriol- (1.2.10)

$R^8$ = Alkyl

4 - Butyl - butanetriol - (1.2.4)
4 - Pentyl - "
4 - Hexyl - "
4 - Heptyl - "
4 - Octyl - "
4 - Tetradecyl - "
4 - Hexadecyl - "
4 - Octadecyl - "
4 - Eicosanyl - "
4 - Erucyl - "

4 - Ethyl - pentanetriol - (1.2.5)
4 - Propyl - "
4 - Butyl - "
4 - Pentyl - "
4 - Hexyl - "
4 - Heptyl - "
4 - Octyl - "

$R^8$ = Acyl

4 - Acetyl - butanetriol - (1.2.4)
4 - Propionyl - "
4 - Butanoyl - "
4 - Pentanoyl - "
4 - Hexanoyl - "

4 - Heptanoyl - "
4 - Octanoyl - "
4 - Nonanoyl - "
4 - Decanoyl - "
4 - Dodecanoyl - "
4 - Myristoyl - "
4 - Palmitoyl - "
4 - Stearoyl - "
4 - Oleoyl - "
4 - Eicosanoyl - "
4 - Docosanoyl - "
4 - Erucoyl - "

5 - Acetyl - pentanetriol - (1.2.5)
5 - Butanoyl - "
5 - Hexanoyl - "
5 - Octanoyl - "
5 - Decanoyl - "
5 - Tetradecanoyl -"
5 - Hexadecanoyl - "
5 - Octadecanoyl - "
5 - Oleoyl - "
5 - Erucoyl - "

6 - O - Hecyl - hexanetriol - (1.2.6) (Ether)

6 - O - Hexanoyl - hexanetriol - (1.2.6) (Ester)

8 - O - ($\alpha$-Hydroxy) - octanoyl - octanetriol - (1.2.8) ($\alpha$-Hydroxyester)

Group D

$R^8$ = alkyl, p = 1, z = 1

Methyl - ethyleneglyko - glycerol
Ethy - "
Propyl - "
Butyl - "
Pentyl - "
Hexyl - "
Heptyl - "
Octyl - "
Nonyl - "
Decyl - "
Undecyl - "
Undecenyl - "
Dodecyl - "
Tetradecyl - "
Hexadecyl - "
Octadecyl - "
Oleyl - "
Eicosanyl - "
Erucyl - "

$R^8$ = Alkyl, p = 2, z = 1

Methyl - di-ethyleneglyko - glycerol
Ethyl - "

Propyl - "
Butyl - "
Pentyl - "
Hexyl - "
Heptyl - "
Octyl - "
Nonyl - "
Decyl - "
Undecyl - "
Undecenyl - "

Dodecyl - "
Tetradecyl - "
Hexadecyl - "
Octadecyl - "
Oleyl - "
Eicosanyl - "
Erucyl - "

$R^8$ = Alkyl, p = 3, z = 1

Methyl - tri-ethyleneglyko - glycerol Ethyl - "
Propyl - "
Butyl - "
Pentyl - "
Hexyl - "
Heptyl - "
Octyl - "
Nonyl - "
Decyl - "
Undecyl - "
Undecenyl - "
Dodecyl - "
Tetradecyl - "
Hexadecyl - "
Octadecyl - "
Oleyl - "
Eicosanyl - "
Erucyl - "

$R^8$ = Alkyl, p = 4, z = 1

Methyl - tetra - ethyleneglyko - glycerol
Ethyl - "
Propyl - "
Butyl - "
Pentyl - "
Hexyl - "
Heptyl - "
Octyl - "
Nonyl - "
Decyl - "
Undecyl - "
Undecenyl - "
Dodecyl - "
Tetradecyl - "
Hexadecyl - "
Octadecyl - "

Oleyl - "
Eicosanyl - "
Erucyl - "

$R^8$ = Alkyl, p = 1, z = 2

Methyl - ethyleneglyko - di - glycerol Ethyl - "
Propyl - "
Butyl - "
Pentyl - "
Hexyl - "
Heptyl - "
Octyl - "
Nonyl - "
Decyl - "
Undecyl - "
Undecenyl - "

Dodecyl - "
Tetradecyl - "
Hexadecyl - "
Octadecyl - "
Oleyl - "
Eicosanyl - "
Erucyl - "

$R^8$ = Alkyl, p = 2, z = 2

Methyl - di-ethyleneglyko - di - glycerol Ethyl - "
Propyl - "
Butyl - "
Pentyl - "
Hexyl - "
Heptyl - "
Octyl - "
Nonyl - "
Decyl - "
Undecyl - "
Undecenyl - "

Dodecyl - "
Tetradecyl - "
Hexadecyl - "
Octadecyl - "
Oleyl - "
Eicosanyl - "
Erucyl - "

$R^8$ = Alkyl, p = 3, z = 2

Methyl - tri-ethyleneglyko - tri - glycerol Ethyl - "
Propyl - "
Butyl - "
Pentyl - "
Hexyl - "
Heptyl - "
Octyl - "
Nonyl - "

Decyl - "
Undecyl - "
Undeceneyl - "

Dodecyl - "
Tetradecyl - "
Hexadecyl - "
Octadecyl - "
Oleyl - "
Eicosanyl - "
Erucyl - "

$R^8$ = Alkyl, p = 4, z = 2

Methyl - tetra - ethyleneglyko - di - glycerol Ethyl - "
Propyl - "
Butyl - "
Pentyl - "
Hexyl - "
Heptyl - "
Octyl - "
Nonyl - "
Decyl - "
Undecyl - "
Undecenyl - "
Dodecyl- "
Tetradecyl - "
Hexadecyl - "
Octadecyl - "
Oleyl - "
Eicosanyl - "
Erucyl - "

$R^8$ = Alkyl, p = 1, z = 3

Methyl - ethyleneglyko - tri - glycerol Ethyl - "
Propyl - "
Butyl - "
Pentyl - "
Hexyl - "
Heptyl - "
Octyl - "
Nonyl - "
Decyl - "
Undecyl - "
Undecenyl - "

Dodecyl - "
Tetradecyl - "
Hexadecyl - "
Octadecyl - "
Oleyl - "
Eicosanyl - "
Erucyl - "

$R^8$ = Alkyl, p = 2, z = 3

Methyl - di - ethyleneglyko - tri - glycerol Ethyl - "

Propyl - "
Butyl - "
Pentyl - "
Hexyl - "
Heptyl - "
Octyl - "
Nonyl - "
Decyl - "
Undecyl - "
Undecenyl - "

Dodecyl - "
Tetradecyl - "
Hexadecyl - "
Octadecyl - "
Oleyl - "
Eicosanyl - "
Erucyl - "

$R^8$ = Alkyl, p = 3, z = 3

Methyl - tri - ethyleneglyko - tri - glycerol Ethyl - "
Propyl - "
Butyl - "
Pentyl - "
Hexyl - "
Heptyl - "
Octyl - "
Nonyl - "
Decyl - "
Undecyl - "
Undecenyl - "
Dodecyl - "
Tetradecyl - "
Hexadecyl - "
Octadecyl - "
Oleyl - "
Eicosanyl - "
Erucyl - "

$R^8$ = Alkyl, p = 4, z = 3

Methyl - tetra - ethyleneglyko - tri - glycerol Ethyl - "
Propyl - "
Butyl - "
Pentyl - "
Hexyl - "
Heptyl - "
Octyl - "
Nonyl - "
Decyl - "
Undecyl - "
Undecenyl - "
Dodecyl - "
Tetradecyl - "
Hexadecyl - "
Octadecyl - "
Oleyl - "

Eicosanyl - "
Erucyl - "

$R^8$ = Acyl, p = 1, z = 1

Acetyl - ethyleneglyko - glycerol Propionyl - "
Butanoyl - "
Pentanoyl - "
Hexanoyl - "
Heptanoyl - "
Octanoyl - "
Nonanoyl - "
Decanoyl - "
Undecanoyl - "
Undecenoyl - "
Dodecanoyl - "
Tetradecanoyl - "
Hexadecanoyl - "
Octadecanoyl - "
Oleoyl - "
Eicosanoyl - "
Erucoyl - "

$R^8$ = Acyl, p = 2, z = 1

Acetyl - di - ethyleneglyko - glycerol Propionyl - "
Butanoyl - "
Pentanoyl - "
Hexanoyl - "
Heptanoyl - "
Octanoyl - "
Nonanoyl - "
Decanoyl - "
Undecanoyl - "
Undecenoyl - "
Dodecanoyl - "
Tetradecanoyl - "
Hexadecanoyl - "
Octadecanoyl - "
Oleoyl - "
Eicosanoyl - "
Erucoyl - "

$R^8$ = Acyl, p = 3, z = 1

Acetyl - tri - ethyleneglyko - glycerol Propionyl - "
Butanoyl - "
Pentanoyl - "
Hexanoyl - "
Heptanoyl - "
Octanoyl - "
Nonanoyl - "
Decanoyl - "
Undecanoyl - "
Undecenoyl - "
Dodecanoyl - "
Tetradecanoyl - "
Hexadecanoyl - "

Octadecanoyl - "
Oleoyl - "
Eicosanoyl - "
Erucoyl - "

$R^8$ = Acyl, p = 4, z = 1

Acetyl - tetra - ethyleneglyko - glycerol Propionyl - "
Butanoyl - "
Pentanoyl - "
Hexanoyl - "
Heptanoyl - "
Octanoyl - "
Nonanoyl - "
Decanoyl - "
Undecanoyl - "
Undecenoyl - "
Dodecanoyl - "
Tetradecanoyl - "
Hexadecanoyl - "
Octadecanoyl - "
Oleoyl - "
Eicosanoyl - "
Erucoyl - "

$R^8$ = Acyl, p = 1, z = 2

Acetyl - ethyleneglyko - di - glycerol Propionyl - "
Butanoyl - "
Pentanoyl - "
Hexanoyl - "
Heptanoyl - "
Octanoyl - "
Nonanoyl - "
Decanoyl - "
Undecanoyl - "
Undecenoyl - "

Dodecanoyl - "
Tetradecanoyl - "
Hexadecanoyl - "
Octadecanoyl - "
Oleoyl - "
Eicosanoyl - "
Erucoyl - "

$R^8$ = Acyl, p = 2, z = 2

Acetyl - di - ethyleneglyko - di - glycerol Propionyl - "
Butanoyl - "
Pentanoyl - "
Hexanoyl - "
Heptanoyl - "
Octanoyl - "
Nonanoyl - "
Decanoyl - "
Undecanoyl - "
Undecenoyl - "

Dodecanoyl - "
Tetradecanoyl - "
Hexadecanoyl - "
Octadecanoyl - "
Oleoyl - "
Eicosanoyl - "
Erucoyl - "

$R^8$ = Acyl, p = 3, z = 2

Acetyl -tri - ethyleneglyko - di - glycerol
Propionyl - "
Butanoyl - "
Pentanoyl - "
Hexanoyl - "
Heptanoyl - "
Octanoyl - "
Nonanoyl - "
Decanoyl - "
Undecanoyl - "
Undecenoyl - "
Dodecanoyl - "
Tetradecanoyl - "
Hexadecanoyl - "
Octadecanoyl - "
Oleoyl - "
Eicosanoyl - "
Erucoyl- "

$R^8$ = Acyl, p = 4, z = 2

Acetyl - tetra - ethyleneglyko - di - glycerol
Propionyl - "
Butanoyl - "
Pentanoyl - "
Hexanoyl - "
Heptanoyl - "
Octanoyl - "
Nonanoyl - "
Decanoyl - "
Undecanoyl - "
Undecenoyl - "
Dodecanoyl - "
Tetradecanoyl - "
Hexadecanoyl - "
Octadecanoyl - "
Oleoyl - "
Eicosanoyl - "
Erucoyl - "

$R^8$ = Acyl, p = 1, z = 3

Acetyl - ethyleneglyko - tri - glycerol Propionyl - "
Butanoyl - "
Pentanoyl - "
Hexanoyl - "
Heptanoyl - "
Octanoyl - "

Nonanoyl - "
Decanoyl - "
Undecanoyl - "
Undecenoyl - "
Dodecanoyl - "
Tetradecanoyl - "
Hexadecanoyl - "
Octadecanoyl - "
Oleoyl - "
Eicosanoyl - "
Erucoyl - "

$R^8$ = Acyl, p = 2, z = 3

Acetyl - di - ethyleneglyko - tri - glycerol Propionyl - "
Butanoyl - "
Pentanoyl - "
Hexanoyl - "
Heptanoyl - "
Octanoyl - "
Nonanoyl - "
Decanoyl - " ,
Undecanoyl - "
Undecenoyl - "
Dodecanoyl - "
Tetradecanoyl - "
Hexadecanoyl - "
Octadecanoyl - "
Oleoyl - "
Eicosanoyl - "
Erucoyl - "

$R^8$ = Acyl, p = 3, z = 3

Acetyl - tri - ethyleneglyko - tri - glycerol Propionyl - "
Butanoyl - "
Pentanoyl - "
Hexanoyl - "
Heptanoyl - "
Octanoyl - "
Nonanoyl - "
Decanoyl - "
Undecanoyl - "
Undecenoyl - "

Dodecanoyl - "
Tetradecanoyl - "
Hexadecanoyl - "
Octadecanoyl - "
Oleoyl - "
Eicosanoyl - "
Erucoyl - "

$R^8$ = Acyl, p = 4, z = 3

Acetyl - tetra - ethyleneglyko - tri - glycerol
Propionyl - "
Butanoyl - "

Pentanoyl - "
Hexanoyl - "
Heptanoyl - "
Octanoyl - "
Nonanoyl - "
Decanoyl - "
Undecanoyl - "
Undecenoyl - "
Dodecanoyl - "
Tetradecanoyl - "
Hexadecanoyl - "
Octadecanoyl - "
Oleoyl - "
Eicosanoyl - "
Erucoyl - "

Group E

$R^8$ = Alkyl, p = 1, z = 1

Methyl - propyleneglyko - glycerol
Ethyl - "
Propyl - "
Butyl - "
Pentyl - "
Hexyl - "
Heptyl - "
Octyl - "
Nonyl - "
Decyl - "
Undecyl - "
Undecenyl - "
Dodecyl - "
Tetradecyl - "
Hexadecyl - "
Octadecyl - "
Oleyl - "
Eicosanyl - "
Erucyl - "

$R^8$ = Alkyl, p = 2, z = 2

Methyl - di - propyleneglyko - glycerol Ethyl - "
Propyl - "
Butyl - "
Pentyl - "
Hexyl - "
Heptyl - "
Octyl - "
Nonyl - "
Decyl - "
Undecyl - "
Undecenyl - "
Dodecyl - "
Tetradecyl - "
Hexadecyl - "
Octadecyl - "
Oleyl - "

Eicosanyl - "
Erucyl - "

$R^8$ = Alkyl, p = 3, z = 1

Methyl - tri - propyleneglyko - glycerol Ethyl - "
Propyl - "
Butyl - "
Pentyl - "
Hexyl - "
Heptyl - "
Octyl - "
Nonyl - "
Decyl - "
Undecyl - "
Undecenyl - "
Dodecyl - "
Tetradecyl - "
Hexadecyl - "
Octadecyl - "
Oleyl - "
Eicosanyl - "
Erucyl - "

$R^8$ = Alkyl, p = 4, z = 1

Methyl - tetra - propyleneglyko - glycerol Ethyl - "
Propyl - "
Butyl - "
Pentyl - "
Hexyl - "
Heptyl - "
Octyl - "
Nonyl - "
Decyl - "
Undecyl - "
Undecenyl - "
Dodecyl - "
Tetradecyl - "
Hexadecyl - "
Octadecyl - "
Oleyl - "
Eicosanyl - "
Erucyl - "

$R^8$ = Alkyl, p = 1, z = 2

Methyl - propyleneglyko - di - glycerol Ethyl - "
Propyl - "
Butyl - "
Pentyl - "
Hexyl - "
Heptyl - "
Octyl - "
Nonyl - "
Decyl - "
Undecyl - "
Undecenyl - "

Dodecyl - "
Tetradecyl - "
Hexadecyl - "
Octadecyl - "
Oleyl - "
Eicosanyl - "
Erucyl - "

$R^8$ = Alkyl, p = 2, z = 2

Methyl - di - propyleneglyko - di - glycerol
Ethyl - "
Propyl - "
Butyl - "
Pentyl - "
Hexyl - "
Heptyl - "
Octyl - "
Nonyl - "
Decyl - "
Undecyl - "
Undecenyl - "
Dodecyl - "
Tetradecyl - "
Hexadecyl - "
Octadecyl - "
Oleyl - "
Eicosanyl - "
Erucyl - "

$R^8$ = Alkyl, p = 3, z = 2

Methyl - tri - propyleneglyko - tri - glycerol
Ethyl - "
Propyl - "
Butyl - "
Pentyl - "
Hexyl - "
Heptyl - "
Octyl - "
Nonyl - "
Decyl - "
Undecyl - "
Undecenyl - "
Dodecyl - "
Tetradecyl - "
Hexadecyl - "
Octadecyl - "
Oleyl - "
Eicosanyl - "
Erucyl - "

$R^8$ = Alkyl, p = 4, z = 2

Methyl - tetra -propyleneglyko - di - glycerol Ethyl - "
Propyl - "
Butyl - "
Pentyl - "

Hexyl - "
Heptyl - "
Octyl - "
Nonyl - "
Decyl - "
Undecyl - "
Undecenyl - "
Dodecyl - "
Tetradecyl - "
Hexadecyl - "
Octadecyl - "
Oleyl - "
Eicosanyl - "
Erucyl - "

$R^8$ = Alkyl, p = 1, z = 3

Methyl - propyleneglyko - tri - glycerol
Ethyl - "
Propyl - "
Butyl - "
Pentyl - "
Hexyl - "
Heptyl - "
Octyl - "
Nonyl - "
Decyl - "
Undecyl - "
Undecenyl - "
Dodecyl - "
Tetradecyl - "
Hexadecyl - "
Octadecyl - "
Oleyl - "
Eicosanyl - "
Erucyl - "

$R^8$ = Alkyl, p = 2, z = 3

Methyl - di - propyleneglyko - tri - glycerol
Ethyl - "
Propyl - "
Butyl- "
Pentyl - "
Hexyl - "
Heptyl - "
Octyl - "
Nonyl - "
Decyl - "
Undecyl - "
Undecenyl - "
Dodecyl - "
Tetradecyl - "
Hexadecyl - "
Octadecyl - "
Oleyl - "
Eicosanyl - "
Erucyl - "

$R^8$ = Alkyl, p = 3, z = 3

Methyl - tri - propyleneglyko - tri - glycerol
Ethyl - "
Propyl - "
Butyl - "
Pentyl - "
Hexyl - "
Heptyl - "
Octyl - "
Nonyl - "
Decyl - "
Undecyl - "
Undecenyl - "
Dodecyl - "
Tetradecyl - "
Hexadecyl - "
Octadecyl - "
Oleyl - "
Eicosanyl - "
Erucyl - "

$R^8$ = Alkyl, p = 4, z = 3

Methyl - tetra - propyleneglyko - tri - glycerol Ethyl - "
Propyl - "
Butyl - "
Pentyl - "
Hexyl - "
Heptyl - "
Octyl - "
Nonyl - "
Decyl - "
Undecyl - "
Undecenyl - "
Dodecyl - "
Tetradecyl - "
Hexadecyl - "
Octadecyl - "
Oleyl - "
Eicosanyl - "
Erucyl - "

$R^8$ = Acyl, p = 1, z = 1

Acetyl - propyleneglyko - glycerol Propionyl - "
Butanoyl - "
Pentanoyl - "
Hexanoyl - "
Heptanoyl - "
Octanoyl - "
Nonanoyl - "
Decanoyl- "
Undecanoyl - "
Undecenoyl - "
Dodecanoyl - "
Tetradecanoyl - "
Hexadecanoyl - " Octadecanoyl - "

Oleoyl - "
Eicosanoyl - "
Erucoyl - "

$R^8$ = Acyl, p = 2, z = 1

Acetyl - di - propyleneglyko - glycerol Propionyl - "
Butanoyl - "
Pentanoyl - "
Hexanoyl - "
Heptanoyl - "
Octanoyl - "
Nonanoyl - "
Decanoyl - "
Undecanoyl - "
Undecenoyl - "
Dodecanoyl - "
Tetradecanoyl - "
Hexadecanoyl - "
Octadecanoyl - "
Oleoyl - "
Eicosanoyl - "
Erucoyl - "

$R^8$ = Acyl, p = 3, z = 1

Acetyl - tri - propyleneglyko - glycerol Propionyl - "
Butanoyl - "
Pentanoyl - "
Hexanoyl - "
Heptanoyl - "
Octanoyl - "
Nonanoyl - "
Decanoyl - "
Undecanoyl - "
Undecenoyl - "
Dodecanoyl - "
Tetradecanoyl - "
Hexadecanoyl - "
Octadecanoyl - "
Oleoyl - "
Eicosanoyl - "
Erucoyl - "

$R^8$ = Acyl, p = 4, z = 1

Acetyl - tetra - propyleneglyko - glycerol Propionyl - "
Butanoyl - "
Pentanoyl - "
Hexanoyl - "
Heptanoyl - "
Octanoyl - "
Nonanoyl - "
Decanoyl - "
Undecanoyl - "
Undecenoyl - "
Dodecanoyl - "
Tetradecanoyl - "

Hexadecanoyl - "
Octadecanoyl - "
Oleoyl - "
Eicosanoyl - "
Erucoyl - "

$R^8$ = Acyl, p = 1, z = 2

Acetyl - propyleneglyko - di - glycerol Propionyl - "
Butanoyl - "
Pentanoyl - "
Hexanoyl - "
Heptanoyl - "
Octanoyl - "
Nonanoyl - "
Decanoyl - "
Undecanoyl - "
Undecenoyl - "
Dodecanoyl - "
Tetradecanoyl - "
Hexadecanoyl - "
Octadecanoyl - "
Oleoyl - "
Eicosanoyl - "
Erucoyl - "

$R^8$ = Acyl, p = 2, z = 2

Acetyl - di - propyleneglyko - di - glycerol Propionyl - "
Butanoyl - "
Pentanoyl - "
Hexanoyl - "
Heptanoyl - "
Octanoyl - "
Nonanoyl - "
Decanoyl - "
Undecanoyl - "
Undecenoyl - "
Dodecanoyl - "
Tetradecanoyl - "
Hexadecanoyl - "
Octadecanoyl - "
Oleoyl - "
Eicosanoyl - "
Erucoyl - "

$R^8$ = Acyl, p = 3, z = 2

Acetyl -tri - propylenglyko - tri - glycerol Propionyl - "
Butanoyl - "
Pentanoyl - "
Hexanoyl - "
Heptanoyl - "
Octanoyl - "
Nonanoyl - "
Decanoyl - "
Undecanoyl - "
Undecenoyl - "

Dodecanoyl - "
Tetradecanoyl - "
Hexadecanoyl - "
Octadecanoyl - "
Oleoyl - "
Eicosanoyl - "
Erucoyl - "

$R^8$ = Acyl, p = 4, z = 4

Acetyl - tetra - propylenglyko - di - glycerol
Propionyl - "
Butanoyl - "
Pentanoyl - "
Hexanoyl - "
Heptanoyl - "
Octanoyl - "
Nonanoyl - "
Decanoyl - "
Undecanoyl - "
Undecenoyl - "
Dodecanoyl - "
Tetradecanoyl - "
Hexadecanoyl - "
Octadecanoyl - "
Oleoyl - "
Eicosanoyl - "
Erucoyl - "

$R^8$ = Acyl, p = 1, z = 2

Acetyl - propyleneglyko - tri - glycerol
Propionyl - "
Butanoyl - "
Pentanoyl - "
Hexanoyl - "
Heptanoyl - "
Octanoyl - "
Nonanoyl - "
Decanoyl - "
Undecanoyl - "
Undecenoyl - "
Dodecanoyl - "
Tetradecanoyl - "
Hexadecanoyl - "
Octadecanoyl - "
oleoyl - "
Eicosanoyl - "
Erucoyl - "

$R^8$ = Acyl, p = 2, z = 3

Acetyl - di - propyleneglyko - tri - glycerol
Propionyl - "
Butanoyl - "
Pentanoyl - "
Hexanoyl - "
Heptanoyl - "

Octanoyl - "
Nonanoyl - "
Decanoyl - "
Undecanoyl - "
Undecenoyl - "
Dodecanoyl - "
Tetradecanoyl - "
Hexadecanoyl - "
Octadecanoyl - "
Oleoyl - "
Eicosanoyl - "
Erucoyl - "

$R^8$ = Acyl, p = 3, z = 3

Acetyl - tri - propyleneglyko- tri - glycerol Propionyl - "
Butanoyl - "
Pentanoyl - "
Hexanoyl - "
Heptanoyl - "
Octanoyl - "
Nonanoyl - "
Decanoyl - "
Undecanoyl - "
Undecenoyl - "
Dodecanoyl - "
Tetradecanoyl - "
Hexadecanoyl - "
Octadecanoyl - "
Oleoyl - "
Eicosanoyl - "
Erucoyl - "

$R^8$ = Acyl, p = 4, z = 3

Acetyl - tetra - propyleneglyko - tri - glycerol Propionyl - "
Butanoyl - "
Pentanoyl - "
Hexanoyl - "
Heptanoyl - "
Octanoyl - "
Nonanoyl - "
Decanoyl - "
Undecanoyl - "
Undecenoyl - "
Dodecanoyl - "
Tetradecanoyl - "
Hexadecanoyl - "
Octadecanoyl - "
Oleoyl - "
Eicosanoyl - "
Erucoyl - "

Group F

1-O-Butyl-ethyleneglyko-propyleneglyko-glycerol
1-Undecenoyl-ethyleneglyko-propyleneglyko-glyceroglycerol

Group G

Decyl-ethyleneglyko-arabitol
Decanoyl-ethyleneglyko-arabitol

## Claims

1. A pharmaceutical composition comprising a compound of formula (I):

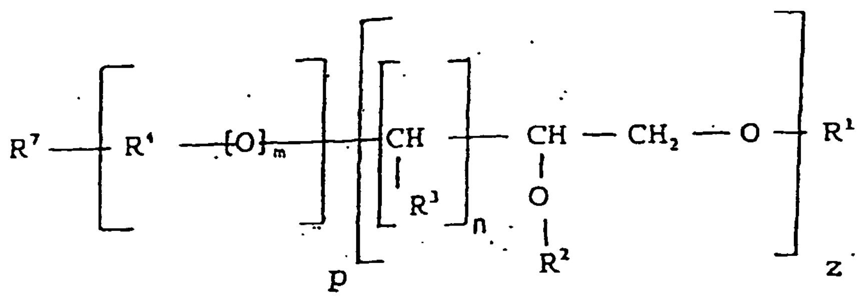

wherein:
$R^1$, $R^2$, $R^6$, $R^8$ and $R^9$ independently at each occurrence represent hydrogen or a linear or branched, saturated or unsaturated,
substituted or unsubstituted hydrocarbon or acyl group, provided that at least one of $R^1$ and $R^2$ is H,
$R^3$ independently at each occurrence represents H, OH or $-O-R^9$,
$R^4$ independently at each occurrence represents $-(CH_2)_x-$ or $-CH_2-[CH(R^5)-]_yCH_2-$,
$R^5$ independently at each occurrence represents H, OH, $R^6$ or $-OR^6$,
$R^7$ represents H, OH, $CH_3$ or $-O-R^8$,
n is an integer from 0 to 6,
m is 0 or 1,
p is an integer from 1 to 9
x is an integer from 0 to 12
y is an integer from 1 to 4 and
z is an integer from 1 to 8.

2. The pharmaceutical composition of claim 1 comprising a compound of formula (II):

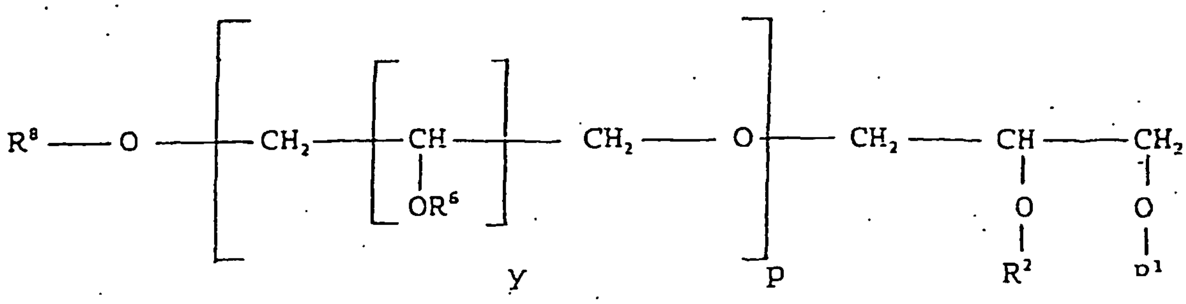

wherein:

$R^1$, $R^2$, $R^6$ and $R^8$ are defined as in claim 1,
y is an integer from 1 to 4; and
p is an integer from 1 to 9.

3. The pharmaceutical composition of claim 1 comprising a compound of formula (III):

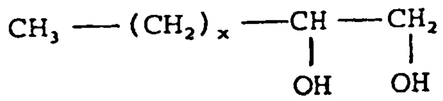

wherein:

x is an integer from 1 to 12.

**4.** The pharmaceutical composition of claim 1 comprising a compound of formula (IV):

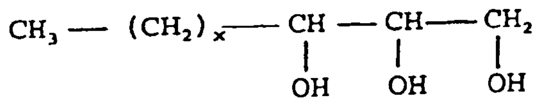

wherein:

x is an integer from 1 to 12.

**5.** The pharmaceutical composition of claim 1 comprising a compound of formula (V):

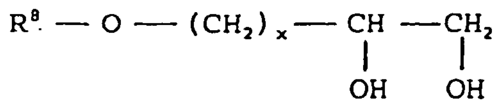

wherein:

$R^8$ is defined as in claim 1 and
x is an integer from 1 to 12.

**6.** The pharmaceutical composition of claim 1 comprising a compound of formula (VI):

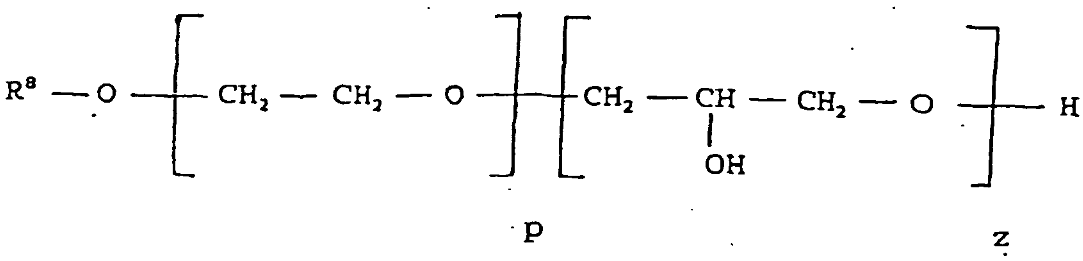

wherein:

$R^8$, p and z are defined as in claim 1.

**7.** The pharmaceutical composition of claim 1 comprising a compound of formula (VII):

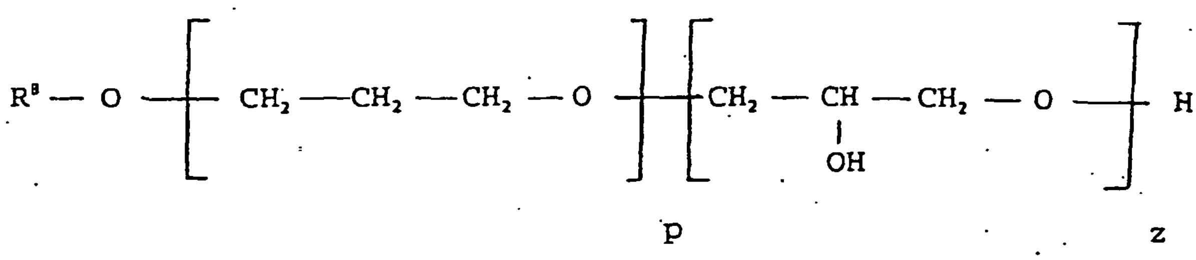

wherein:

$R^8$, p and z are defined as in claim 1.

**8.** The pharmaceutical composition of claim 1 comprising a compound of formula (VIII):

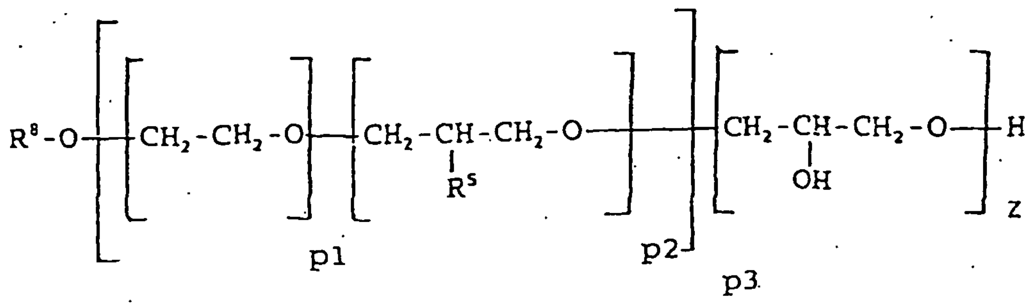

wherein:

$R^8$, $R^5$ and z are defined as in claim 1 and
p1 is an integer from 0 to 20,
p2 is an integer from 0 to 20 and
p3 is an integer from 1 to 10,
with the proviso that p 1 + p2 ≥ 1 and with the proviso that, if p1 = 0 at least one $R^5$ = H.

**9.** The pharmaceutical composition of claim 1 comprising a compound of formula (IX):

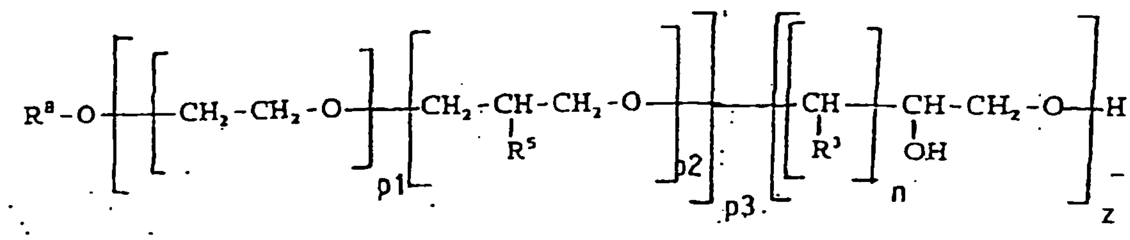

wherein:

$R^3$, $R^5$, $R^8$ and z are defined as in claim 1 and
p1 is an integer from 0 to 20,
p2 is an integer from 0 to 20,
p3 is an integer from 1 to 10 and
n is an integer ≥ 2.

**10.** The pharmaceutical composition of any of the preceding claims, wherein:

a) $R^1$ and $R^2$ are independently a $C_1$-$C_{22}$ alkyl, alkenyl, alkynyl or acyl group, provided that one of $R^1$ or $R^2$ is -H;
b) each $R^5$ is -H or a $C_1$-$C_{22}$ alkyl, alkenyl, alkynyl or acyl group, and is independently selected; and
c) p is an integer from 1 to 6.

**11.** The pharmaceutical composition of any one of the preceding claims, wherein each $R^6$ is -H.

**12.** The pharmaceutical composition of any one of the preceding claims, wherein y is 1.

**13.** The pharmaceutical composition of any one of the preceding claims, wherein $R^1$ is $C_4$-$C_{12}$ alkyl and $R^2$ is -H.

**14.** The pharmaceutical composition of any one of the preceding claims, wherein p is 2 or 3.

**15.** The pharmaceutical composition of claim 1, wherein the compound is 3-(3-hexyloxy-2-hydroxy-propoxy)-propane-1, 2-diol or
3-[2-hydroxy-3-(2-hydroxy-2-octyloxy-propoxy)-propoxy]-propane-1,2-diol.

**16.** The pharmaceutical composition of any one of the preceding claims further comprising a pharmaceutically active agent.

**17.** The pharmaceutical composition of any one of the preceding claims further comprising common pharmaceutical additives and/or diluents.

**18.** The pharmaceutical composition of claim 16 or 17, wherein the pharmaceutically active agent is a medicament for treating disorders of the brain.

**19.** The pharmaceutical composition of claim 16 or 17, wherein the pharmaceutically active agent is a medicament for treating disorders of the gastrointestinal tract.

**20.** The pharmaceutical composition of claim 16 or 17, wherein the pharmaceutically active agent is a medicament for treating disorders of the skin.

**21.** The pharmaceutical composition of claim 16 or 17, wherein the pharmaceutically active agent is a medicament for treating a respiratory disorder.

**22.** Use of a compound represented by formula (I):

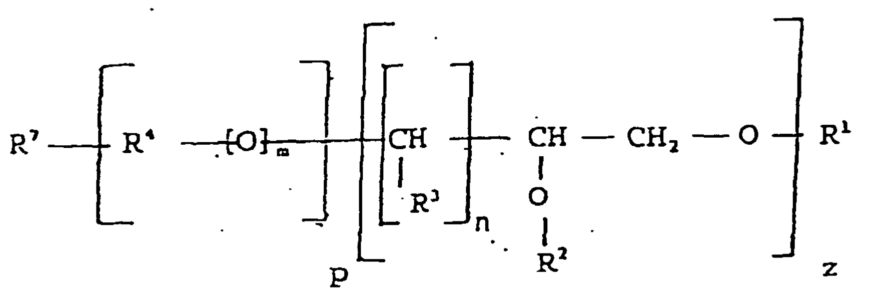

wherein:

$R^1$, $R^2$, $R^6$, $R^8$ and $R^9$ independently at each occurrence represent hydrogen or a linear or branched, saturated or unsaturated, substituted or unsubstituted hydrocarbon or acyl group, provided that at least one of $R^1$ and $R^2$ is H,
$R^3$ independently at each occurrence represents H, OH or -O-$R^9$, $R^4$ independently at each occurrence represents -$(CH_2)_x$- or -$CH_2$-[CH($R^5$)-]$_y$$CH_2$-,
$R^5$ independently at each occurrence represents H, OH, $R^6$ or -O$R^6$,
$R^7$ represents H, OH, $CH_3$ or -O-$R^8$,
n is an integer from 0 to 6,
m is 0 or 1,

p is an integer from 1 to 9,
x is an integer from 0 to 1 2 ,
y is an integer from 1 to 4 a nd
z is an integer from 1 to 8

for the manufacture or a pharmaceutical composition for delivering a pharmaceutically active agent to the brain.

**23.** The use of claim 22, wherein the pharmaceutical composition is for intravenous or intraarterial administration.

**24.** Use of a compound represented by formula (I):

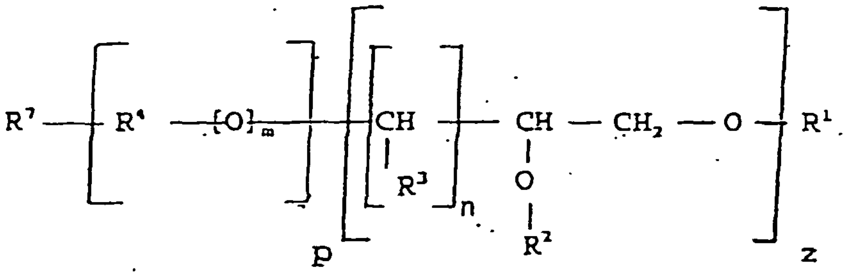

wherein:

$R^1$, $R^2$, $R^6$, $R^8$ and $R^9$ independently at each occurrence represent hydrogen or a linear or branched, saturated or unsaturated, substituted or unsubstituted hydrocarbon or acyl group, provided that at least one of $R^1$ and $R^2$ is H,
$R^3$ independently at each occurrence represents H, OH or -O-$R^9$,
$R^4$ independently at each occurrence represents $-(CH_2)_x-$ or $-CH_2-[CH(R^5)-]_yCH_2-$,
$R^5$ independently at each occurrence represents H, OH, $R^6$ or -O-$R^6$,
$R^7$ represents H, OH, $CH_3$ or -O-$R^8$,
n is an integer from 0 to 6,
m is 0 or 1,
p is an integer from 1 to 9,
x is an integer from 0 to 12,
y is an integer from 1 to 4 and
z is an integer from 1 to - 8

for the manufacture of a pharmaceutical composition for delivering a pharmaceutically active agent to the gastrointestinal tract.

**25.** The use of claim 24, wherein the pharmaceutical composition is encapsulated and administered orally.

**26.** Use of a compound represented by formula (I):

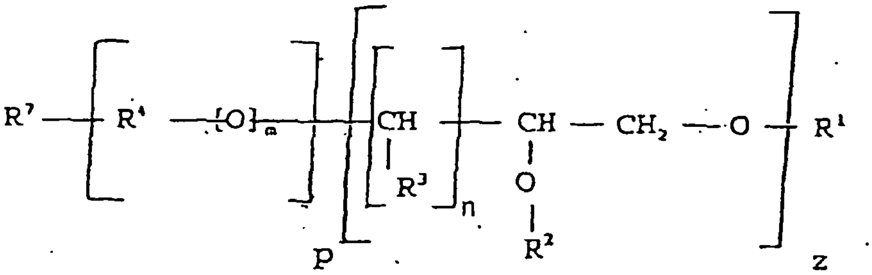

wherein:

$R^1$, $R^2$, $R^6$, $R^8$ and $R^9$ independently at each occurrence represent hydrogen or a linear or branched, saturated or unsaturated, substituted or unsubstituted hydrocarbon or acyl group, provided that at least one of $R^1$ and $R^2$ is H,
$R^3$ independently at each occurrence represents H, OH or $-O-R^9$,
$R^4$ independently at each occurrence represents $-(CH_2)_x-$ or $-CH_2-[CH(R^5)-]_yCH_2-$,
$R^5$ independently at each occurrence represents H, OH, $R^6$ or $-O-R^6$,
$R^7$ represents H, OH, $CH_3$ or $-O-R^8$,
n is an integer from 0 to 6,
m is 0 or 1,
p is an integer from 1 to 9,
x is an integer from 0 to 1 2 ,
y is an integer from 1 to 4 and
z is an integer from 1 to 8

for the manufacture of a pharmaceutical composition for delivering a pharmaceutically active agent to the skin.

**27.** Use of a compound represented by formula (I):

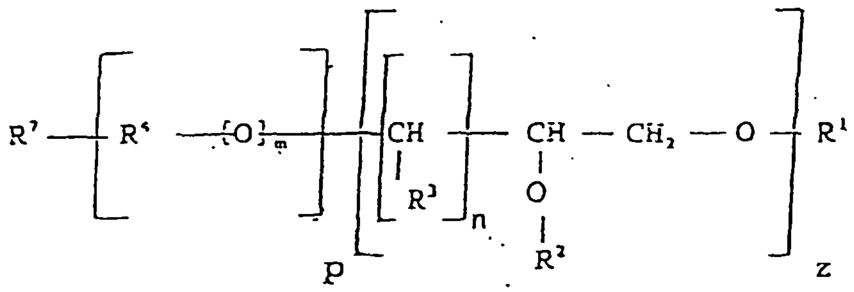

wherein:

$R^1$, $R^2$, $R^6$, $R^8$ and $R^9$ independently at each occurrence represent hydrogen or a linear or branched, saturated or unsaturated, substituted or unsubstituted hydrocarbon or acyl group, provided that at least one of $R^1$ and $R^2$ is H,
$R^3$ independently at each occurrence represents H, OH or $-O-R^9$,
$R^4$ independently at each occurrence represents $-(CH_2)_x-$ or $-CH_2-[CH(R^5)-]_yCH_2-$,
$R^5$ independently at each occurrence represents H, OH, $R^6$ or $-O-R^6$,
$R^7$ represents H, OH, $CH_3$ or $-O-R^8$,
n is an integer from 0 to 6,
m is 0 or 1,
p is an integer from 1 to 9,
x is an integer from 0 to 12,
y is an integer from 1 to 4 and
z is an integer from 1 to 8

for the manufacture of a pharmaceutical composition for delivering a pharmaceutically active agent to the lungs.

**28.** The use according to any one of claims 22-27, wherein the pharmaceutical composition comprises a compound of formula (II):

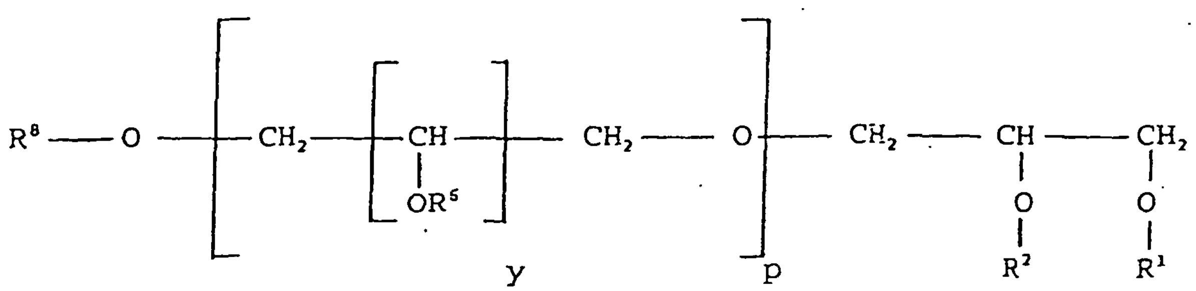

wherein:

$R^1$ and $R^2$ are independently H or a substituted or unsubstituted aliphatic group or a substituted acyl group, provided that one of $R^1$ or $R^2$ is -H,
each $R^6$ is -H or a substituted or unsubstituted aliphatic group or a substituted or unsubstituted acyl group and is independently selected;
y is an integer from 1 to 4, and
p is an integer from 1 to 9.

**29.** The use of claim 28 wherein:

a) $R^1$ and $R^2$ are independently a $C_1$-$C_{22}$ alkyl, alkenyl, alkynyl or acyl group, provided that one of $R^1$ or $R^2$ is -H;
b) each $R^6$ is -H or a $C_1$-$C_{22}$ alkyl, alkenyl, alkynyl or acyl group, and is independently selected; and
c) p is an integer from 1 to 6.

**30.** The use of claim 28 or 29, wherein y is 1 and each $R^6$ is -H.

**31.** The use of any of claims 28-30, wherein p is 2 or 3.

**32.** The use of any of claim 28-31, wherein the compound is
3-(3-hexyloxy-2-hydroxy-propoxy)-propane-1,2-diol or
3-[2-hydroxy-3-(2-hydroxy-2-octyloxy-propoxy)-propoxy]-propane-1,2-diol.

**33.** The use of claim 27, wherein the composition is for administration by pulmonary means.

**34.** Use of a compound of the general formula (I) as defined in claim 1 for the manufacture of a pharmaceutical composition to improve tissue penetration of drugs.

**35.** Use as claimed in claim 34 for the manufacture of a pharmaceutical composition to improve the transport of active substances through the blood-brain barrier.

**36.** Use as claimed in claim 34 for the manufacture of a pharmaceutical composition to improve the resorption of active substances in the gastro-intestinal tract.

**37.** Use as claimed in claim 34 for the manufacture of a pharmaceutical composition to improve penetration of active substances into the skin.

**38.** Use of a compound of the general formula as defined in claim 1 as a solubility mediator for active substances in pharmaceutical preparations.

**39.** Use of a compound of the general formula as defined in claim 1 as an additive to produce liposomal formulations.

**40.** Use as claimed in claim 39, wherein the liposomal formulations are used as pharmaceutical preparations.

## Patentansprüche

1. Pharmazeutische Zusammensetzung, umfassend eine Verbindung der Formel (I)

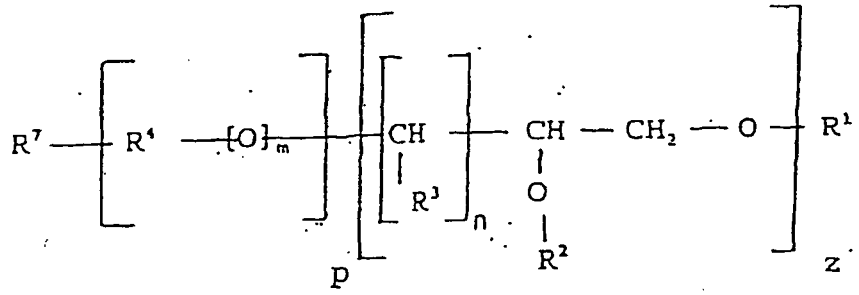

worin:

$R^1$, $R^2$, $R^6$, $R^8$ und $R^9$ unabhängig bei jedem Auftreten Wasserstoff oder eine lineare oder verzweigte, gesättigte oder ungesättigte, substituierte oder unsubstituierte Kohlenwasserstoff- oder Acylgruppe darstellen, mit der Maßgabe, dass mindestens eines von $R^1$ und $R^2$ H ist,
$R^3$ unabhängig bei jedem Auftreten H, OH oder $-O-R^9$ darstellt,
$R^4$ unabhängig bei jedem Auftreten $-(CH_2)_x-$ oder $-CH_2-[CH(R^5)-]yCH_2$ bedeutet,
$R^5$ unabhängig bei jedem Auftreten H, OH, $R^6$ oder $-O-R^6-$ bedeutet,
$R^7$ H, OH, $CH_3$ oder-$O-R^8$ bedeutet,
n eine ganze Zahl von 0 bis 6 ist,
m 0 oder 1 ist,
p eine ganze Zahl von 1 bis 9 ist,
x eine ganze Zahl von 0 bis 12 ist,
y eine ganze Zahl von 1 bis 4 ist und
z eine ganze Zahl von 1 bis 8 ist.

2. Pharmazeutische Zusammensetzung nach Anspruch 1, umfassend eine Verbindung der Formel (II):

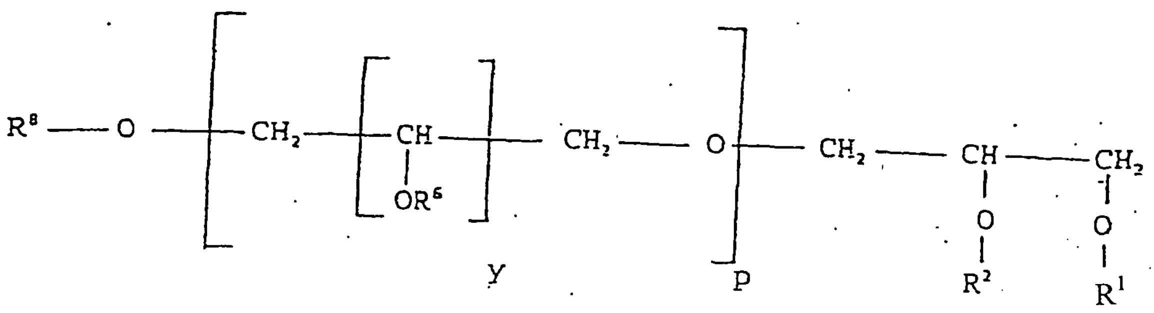

worin:

$R^1$, $R^2$, $R^6$ und $R^8$ wie in Anspruch 1 definiert sind,
y eine ganze Zahl von 1 bis 4 ist; und
p eine ganze Zahl von 1 bis 9 ist.

3. Pharmazeutische Zusammensetzung nach Anspruch 1, umfassend eine Verbindung der Formel (III):

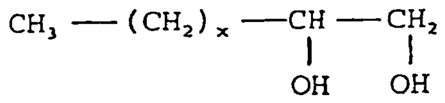

worin:

x eine ganze Zahl von 1 bis 12 ist.

**4.** Pharmazeutische Zusammensetzung nach Anspruch 1, umfassend eine Verbindung der Formel (IV):

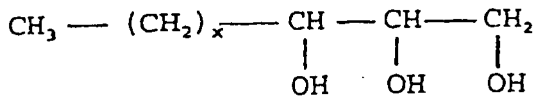

worin:

x eine ganze Zahl von 1 bis 12 ist.

**5.** Pharmazeutische Zusammensetzung nach Anspruch 1, umfassend eine Verbindung der Formel (V):

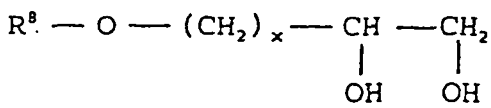

worin:

$R^8$ wie in Anspruch 1 definiert ist und
x eine ganze Zahl von 1 bis 12 ist.

**6.** Pharmazeutische Zusammensetzung nach Anspruch 1, umfassend eine Verbindung der Formel (VI):

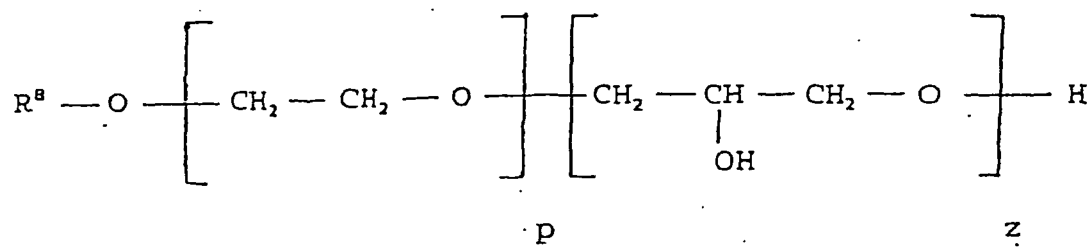

worin:

$R^8$, p und z wie in Anspruch 1 definiert sind.

**7.** Pharmazeutische Zusammensetzung nach Anspruch 1, umfassend eine Verbindung der Formel (VII):

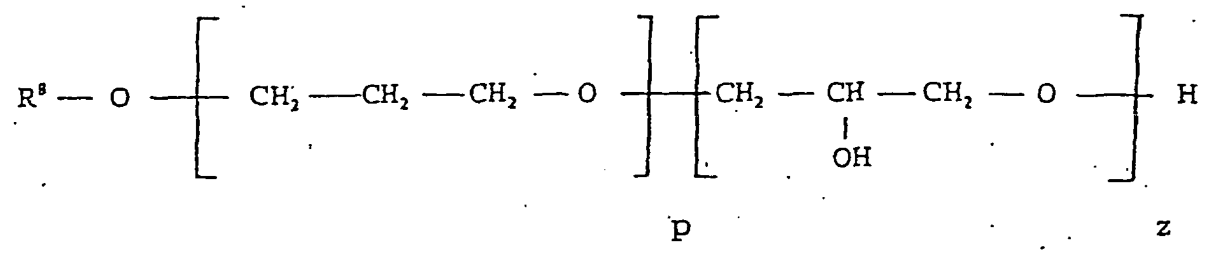

worin:

$R^8$, p und z wie in Anspruch 1 definiert sind.

**8.** Pharmazeutische Zusammensetzung nach Anspruch 1, umfassend eine Verbindung der Formel (VIII):

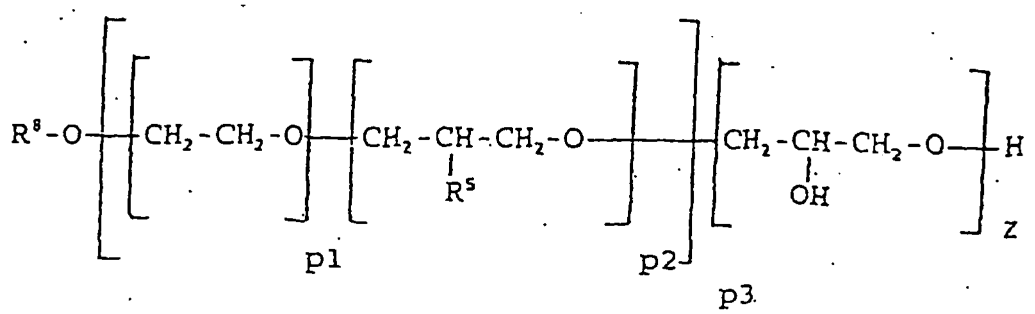

worin:

$R^8$, $R^5$ und z wie in Anspruch 1 definiert sind und
p1 eine ganze Zahl von 0 bis 20 ist,
p2 eine ganze Zahl von 0 bis 20 ist und
p3 eine ganze Zahl von 1 bis 10 ist,

mit der Maßgabe, dass $p1 + p2 \geq 1$ und mir der Maßgabe, dass wenn $p1 = 0$ mindestens ein $R^5 = H$.

**9.** Pharmazeutische Zusammensetzung nach Anspruch 1, umfassend eine Verbindung der Formel (IX):

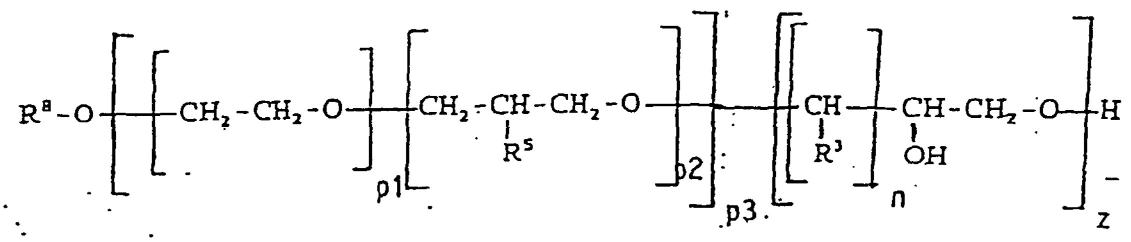

worin:

$R^3$, $R^5$, $R^8$ und z wie in Anspruch 1 definiert sind und
p1 eine ganze Zahl von 0 bis 20 ist,
p2 eine ganze Zahl von 0 bis 20 ist,
p3 eine ganze Zahl von 1 bis 10 ist und
n eine ganze Zahl $\geq 2$ ist.

**10.** Pharmazeutische Zusammensetzung nach einem der vorhergehenden Ansprüche, worin:

a) $R^1$ und $R^2$ unabhängig eine $C_1$-$C_{22}$-Alkyl-, -Alkenyl-, -Alkinyl- oder- Acylgruppe sind, mit der Maßgabe, dass eines aus $R^1$ oder $R^2$ -H ist;
b) jedes $R^6$ -H oder eine $C_1$-$C_{22}$-Alkyl-, -Alkenyl-, -Alkinyl- oder -Acylgruppe ist und unabhängig ausgewählt ist; und
c) p eine ganze Zahl von 1 bis 6 ist.

**11.** Pharmazeutische Zusammensetzung nach einem der vorhergehenden Ansprüche, worin jedes $R^6$ -H ist.

**12.** Pharmazeutische Zusammensetzung nach einem der vorhergehenden Ansprüche, worin y 1 ist.

**13.** Pharmazeutische Zusammensetzung nach einem der vorhergehenden Ansprüche, worin $R^1$ $C_4$-$C_{12}$-Alkyl ist und $R^2$ H ist.

**14.** Pharmazeutische Zusammensetzung nach einem der vorhergehenden Ansprüche, worin p 2 oder 3 ist.

**15.** Pharmazeutische Zusammensetzung nach Anspruch 1, worin die Verbindung 3-(3-Hexyloxy-2-hydroxy-propoxy) propan-1,2-diol oder 3-[2-hydroxy-3-(2-hydroxy-2-octyloxy-propoxy)-propoxy]-propan-1,2-diol ist.

**16.** Pharmazeutische Zusammensetzung nach einem der vorhergehenden Ansprüche, weiterhin umfassend ein pharmazeutisch aktives Mittel.

**17.** Pharmazeutische Zusammensetzung nach einem der vorhergehenden Ansprüche, weiterhin umfassend übliche pharmazeutische Additive und/oder Verdünnungsmittel.

**18.** Pharmazeutische Zusammensetzung nach Anspruch 16 oder 17, worin das pharmazeutisch aktive Mittel ein Medikament zur Behandlung von Erkrankungen des Gehirns ist.

**19.** Pharmazeutische Zusammensetzung nach Anspruch 16 oder 17, worin das pharmazeutisch aktive Mittel ein Medikament zur Behandlung von Erkrankungen des Gastrointestinaltrakts ist.

**20.** Pharmazeutische Zusammensetzung nach Anspruch 16 oder 17, worin das pharmazeutisch aktive Mittel ein Medikament zur Behandlung von Erkrankungen der Haut ist.

**21.** Pharmazeutische Zusammensetzung nach Anspruch 16 oder 17, worin das pharmazeutisch aktive Mittel ein Medikament zur Behandlung von Erkrankungen der Atemwege ist.

**22.** Verwendung einer Verbindung, die durch die Formel (I) dagestellt wird:

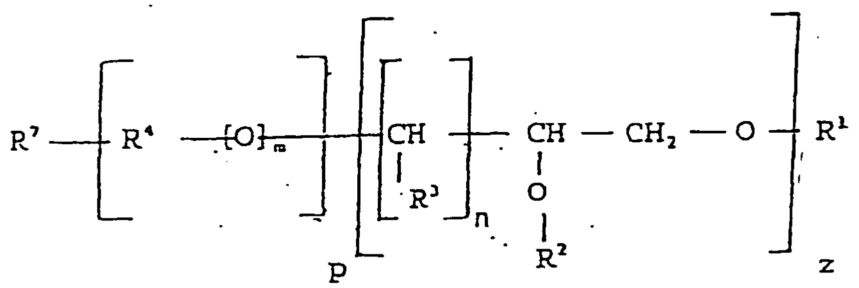

worin:

$R^1$, $R^2$, $R^6$, $R^8$ und $R^9$ unabhängig bei jedem Auftreten Wasserstoff oder eine lineare oder verzweigte, gesättigte oder ungesättigte, substituierte oder unsubstituierte Kohlenwasserstoff- oder Acylgruppe darstellen, mit der Maßgabe, dass mindestens eines von $R^1$ und $R^2$ H ist,
$R^3$ unabhängig bei jedem Auftreten H, OH oder -O-$R^9$ darstellt,
$R^4$ unabhängig bei jedem Auftreten -$(CH_2)_x$- oder -$CH_2$-[CH($R^5$)-]$_y$$CH_2$-bedeutet,
$R^5$ unabhängig bei jedem Auftreten H, OH, $R^6$ oder -O-$R^6$- bedeutet,

$R^7$ H, OH, $CH_3$ oder-O-$R^8$ bedeutet,
n eine ganze Zahl von 0 bis 6 ist,
m 0 oder 1 ist,
p eine ganze Zahl von 1 bis 9 ist,
x eine ganze Zahl von 0 bis 12 ist,
y eine ganze Zahl von 1 bis 4 ist und
z eine ganze Zahl von 1 bis 8 ist,

zur Herstellung einer pharmazeutischen Zusammensetzung, um ein pharmazeutisch wirksames Mittel dem Gehirn zuzuführen.

**23.** Verwendung nach Anspruch 22, worin die pharmazeutische Zusammensetzung für intravenöse oder intraarterielle Verabreichung ist.

**24.** Verwendung einer Verbindung, die durch die Formel (I) dargestellt wird:

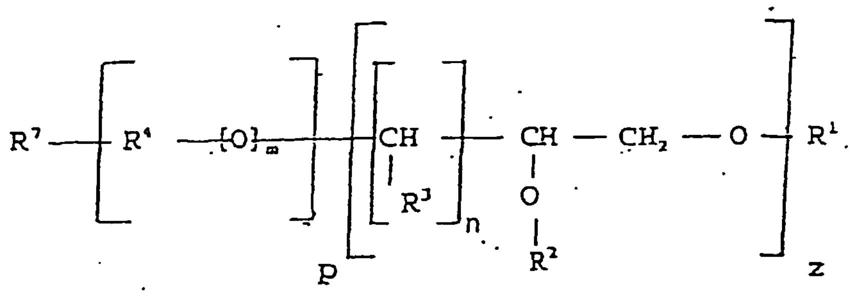

worin:

$R^1$, $R^2$, $R^6$, $R^8$ und $R^9$ unabhängig bei jedem Auftreten Wasserstoff oder eine lineare oder verzweigte, gesättigte oder ungesättigte, substituierte oder unsubstituierte Kohlenwasserstoff- oder Acylgruppe darstellen, mit der Maßgabe, dass mindestens eines von $R^1$ und $R^2$ H ist,
$R^3$ unabhängig bei jedem Auftreten H, OH oder -O-$R^9$ darstellt,
$R^4$ unabhängig bei jedem Auftreten $-(CH_2)_x-$ oder $-CH_2-[CH(R^5)-]_yCH_2-$bedeutet,
$R^5$ unabhängig bei jedem Auftreten H, OH, $R^6$ oder -O-$R^6$- bedeutet,
$R^7$ H, OH, $CH_3$ oder-O-$R^8$ bedeutet,
n eine ganze Zahl von 0 bis 6 ist,
m 0 oder 1 ist,
p eine ganze Zahl von 1 bis 9 ist,
x eine ganze Zahl von 0 bis 12 ist,
y eine ganze Zahl von 1 bis 4 ist und
z eine ganze Zahl von 1 bis 8 ist,

zur Herstellung einer pharmazeutischen Zusammensetzung, um ein pharmazeutisch wirksames Mittel dem Gastrointestinaltrakt zuzuführen.

**25.** Verwendung nach Anspruch 24, worin die pharmazeutische Zusammensetzung eingekapselt ist und oral verabreicht wird.

**26.** Verwendung einer Verbindung, die durch die Formel (I) dargestellt wird:

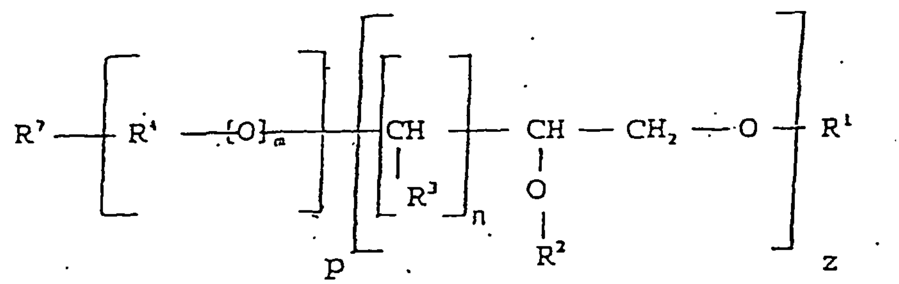

worin:

$R^1$, $R^2$, $R^6$, $R^8$ und $R^9$ unabhängig bei jedem Auftreten Wasserstoff oder eine lineare oder verzweigte, gesättigte oder ungesättigte, substituierte oder unsubstituierte Kohlenwasserstoff- oder Acylgruppe darstellen, mit der Maßgabe, dass mindestens eines von $R^1$ und $R^2$ H ist,
$R^3$ unabhängig bei jedem Auftreten H, OH oder -O-$R^9$ darstellt,
$R^4$ unabhängig bei jedem Auftreten $-(CH_2)_x-$ oder $-CH_2-[CH(R^5)-]_yCH_2-$bedeutet,
$R^5$ unabhängig bei jedem Auftreten H, OH, $R^6$ oder -O-$R^6$- bedeutet,
$R^7$ H, OH, $CH_3$ oder-O-$R^8$ bedeutet,
n eine ganze Zahl von 0 bis 6 ist,
m 0 oder 1 ist,
p eine ganze Zahl von 1 bis 9 ist,
x eine ganze Zahl von 0 bis 12 ist,
y eine ganze Zahl von 1 bis 4 ist und
z eine ganze Zahl von 1 bis 8 ist,

zur Herstellung einer pharmazeutischen Zusammensetzung, um ein pharmazeutisch wirksames Mittel der Haut zuzuführen.

**27.** Verwendung einer Verbindung, die durch die Formel (I) dargestellt wird:

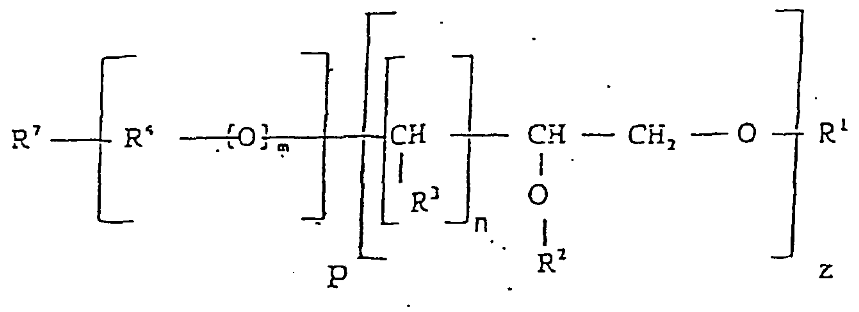

worin:

$R^1$, $R^2$, $R^6$, $R^8$ und $R^9$ unabhängig bei jedem Auftreten Wasserstoff oder eine lineare oder verzweigte, gesättigte oder ungesättigte, substituierte oder unsubstituierte Kohlenwasserstoff- oder Acylgruppe darstellen, mit der Maßgabe, dass mindestens eines von $R^1$ und $R^2$ H ist,
$R^3$ unabhängig bei jedem Auftreten H, OH oder -O-$R^9$ darstellt,
$R^4$ unabhängig bei jedem Auftreten $-(CH_2)_x-$ oder $-CH_2-[CH(R^5)-]_yCH_2-$bedeutet,
$R^5$ unabhängig bei jedem Auftreten H, OH, $R^6$ oder -O-$R^6$- bedeutet,
$R^7$ H, OH, $CH_3$ oder-O-$R^8$ bedeutet,
n eine ganze Zahl von 0 bis 6 ist,
m 0 oder 1 ist,
p eine ganze Zahl von 1 bis 9 ist,
x eine ganze Zahl von 0 bis 12 ist,

y eine ganze Zahl von 1 bis 4 ist und
z eine ganze Zahl von 1 bis 8 ist,

zur Herstellung einer pharmazeutischen Zusammensetzung, um ein pharmazeutisch wirksames Mittel der Lunge zuzuführen.

**28.** Verwendung nach einem der Ansprüche 22-27, worin die pharmazeutische Zusammensetzung eine Verbindung der Formel (II) umfasst:

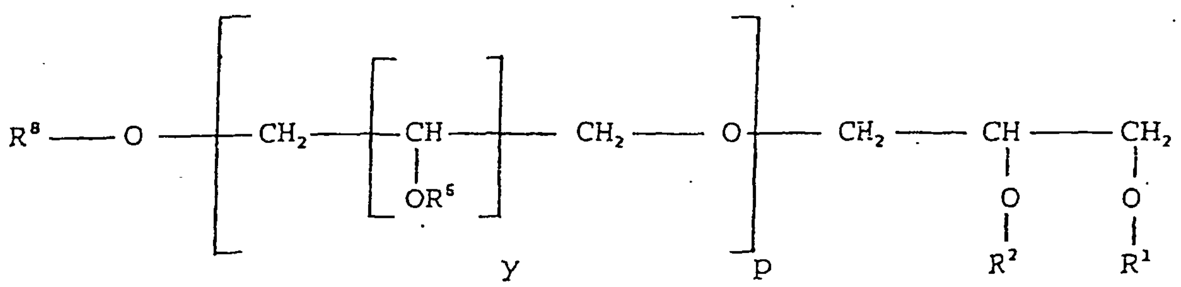

worin:

$R^1$ und $R^2$ unabhängig H oder eine substituierte oder unsubstituierte aliphatische Gruppe oder eine substituierte Acylgruppe sind, mit der Maßgabe, dass eines aus $R^1$ oder $R^2$ -H ist,
jedes $R^6$ -H oder eine substituierte oder unsubstituierte aliphatische Gruppe oder eine substituierte oder unsubstituierte Acylgruppe ist und unabhängig ausgewählt ist;
y eine ganze Zahl von 1 bis 4 ist und
p eine ganze Zahl von 1 bis 9 ist.

**29.** Verwendung nach Anspruch 28, worin:

a) $R^1$ und $R^2$ unabhängig eine $C_1$-$C_{22}$-Alkyl-, -Alkenyl-, -Alkinyl- oder -Acylgruppe sind, mit der Maßgabe, dass eines aus $R^1$ oder $R^2$ -H ist;
b) jedes $R^6$ -H oder eine $C_1$-$C_{22}$-Alkyl-, -Alkenyl-, -Alkinyl- oder -Acylgruppe ist und unabhängig ausgewählt ist; und
c) p eine ganze Zahl von 1 bis 6 ist.

**30.** Verwendung nach Anspruch 28 oder 29, worin y 1 ist und jedes $R^6$ -H ist.

**31.** Verwendung nach einem der Ansprüche 28-30, worin p 2 oder 3 ist.

**32.** Verwendung nach einem der Ansprüche 28-31, worin die Verbindung 3-(3-Hexyloxy-2-hydroxy-propoxy)propan-1,2-diol oder 3-[2-hydroxy-3-(2-hydroxy-2-octyloxy-propoxy)-propoxy]-propan-1,2-diol ist.

**33.** Verwendung nach Anspruch 27, worin die Zusammensetzung zur Verabreichung durch pulmonale Mittel vorgesehen ist.

**34.** Verwendung einer Verbindung der allgemeinen Formel (I) wie in Anspruch 1 definiert zur Herstellung einer pharmazeutischen Zusammensetzung zur Verbesserung der Gewebepenetration von Arzneimitteln.

**35.** Verwendung nach Anspruch 34 zur Herstellung einer pharmazeutischen Zusammensetzung zum Verbessern des Transports von aktiven Substanzen durch die Blut-Hirn-Barriere.

**36.** Verwendung nach Anspruch 34 zur Herstellung einer pharmazeutischen Zusammensetzung zum Verbessern der Resorption aktiver Substanzen in den Gastrointestinaltrakt.

**37.** Verwendung nach Anspruch 34 zur Herstellung einer pharmazeutischen Zusammensetzung zum Verbessern der Penetration aktiver Substanzen in die Haut.

**38.** Verwendung einer Verbindung der allgemeinen Formel nach Anspruch 1 als ein Lösungsvermittler für aktive Substanzen in pharmazeutischen Präparaten.

**39.** Verwendung einer Verbindung der allgemeinen Formel nach Anspruch 1 als Additiv zum Herstellen von Liposomenformulierungen.

**40.** Verwendung nach Anspruch 39, worin die Liposomenformulierungen als pharmazeutische Präparate verwendet werden.

## Revendications

**1.** Composition pharmaceutique comprenant un composé de formule (I) :

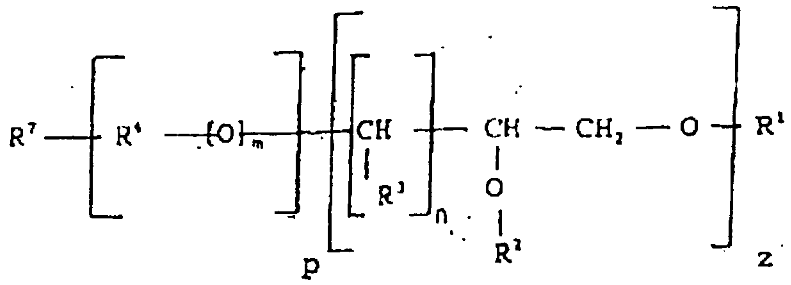

dans laquelle
$R^1$, $R^2$, $R^6$, $R^8$ et $R^9$ indépendamment à chaque occurrence représentent un hydrogène ou un groupe acyle ou hydrocarbure substitué ou non substitué, saturé ou insaturé, ramifié ou linéaire, à condition qu'au moins un parmi $R^1$ et $R^2$ soit H,
$R^3$ indépendamment à chaque occurrence représente H, OH ou $-O-R^9$,
$R^4$ indépendamment à chaque occurrence représente $-(CH_2)_x-$ ou $-CH_2-[CH(R^5)-]_yCH_2-$,
$R^5$ indépendamment à chaque occurrence représente H, OH, $R^6$ ou $-O-R^6$,
$R^7$ représente H, OH, $CH_3$ ou $-O-R^8$,
n est un entier de 0 à 6,
m est 0 ou 1,
p est un entier de 1 à 9,
x est un entier de 0 à 12,
y est un entier de 1 à 4 et
z est un entier de 1 à 8.

**2.** Composition pharmaceutique selon la revendication 1 comprenant un composé de formule (II) :

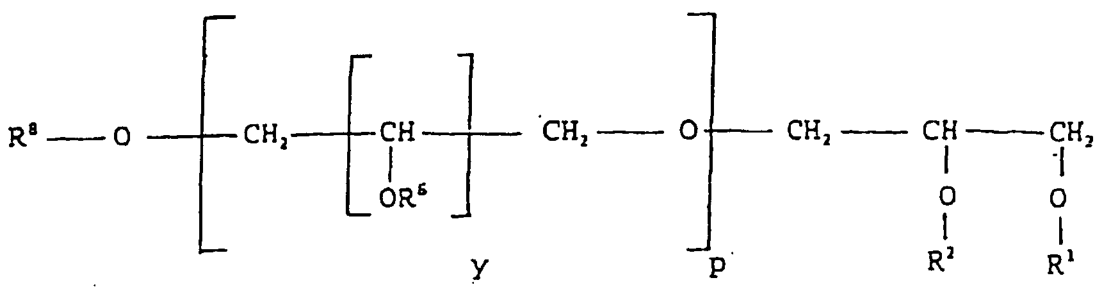

dans laquelle
$R^1$, $R^2$, $R^6$ et $R^8$ sont définis comme dans la revendication 1,
y est un entier de 1 à 4 ; et

p est un entier de 1 à 9.

3. Composition pharmaceutique selon la revendication 1 comprenant un composé de formule (III) :

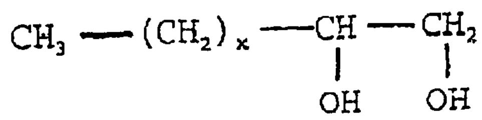

dans laquelle
x est un entier de 1 à 12.

4. Composition pharmaceutique selon la revendication 1 comprenant un composé de formule (IV) :

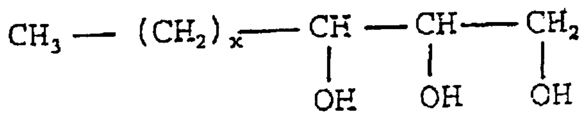

dans laquelle
x est un entier de 1 à 12.

5. Composition pharmaceutique selon la revendication 1 comprenant un composé de formule (V) :

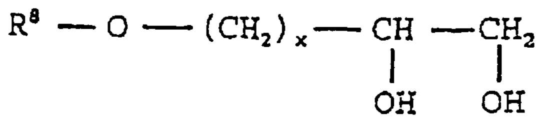

dans laquelle
$R^8$ est défini comme dans la revendication 1 et
x est un entier de 1 à 12.

6. Composition pharmaceutique selon la revendication 1 comprenant un composé de formule (VI) :

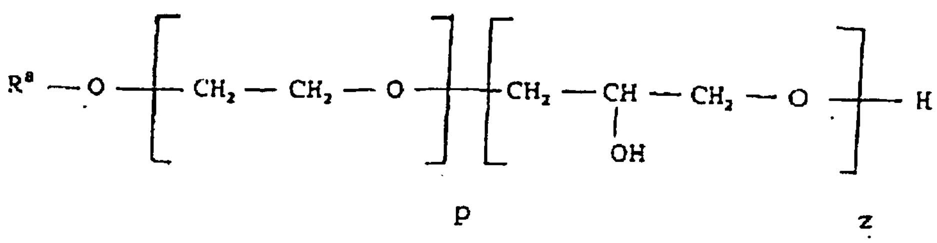

dans laquelle
$R^8$, p et z sont définis comme dans la revendication 1.

7. Composition pharmaceutique selon la revendication 1 comprenant un composé de formule (VII) :

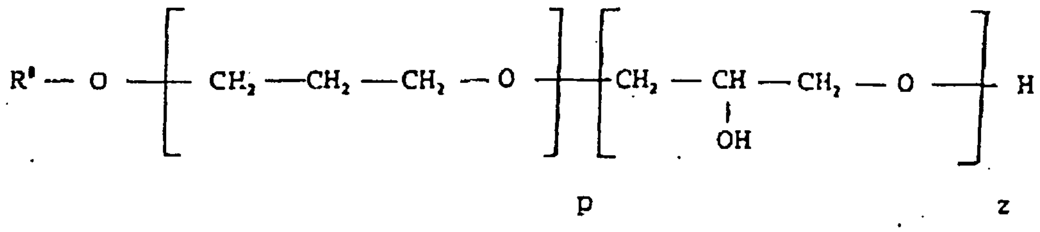

dans laquelle
$R^8$, p et z sont définis comme dans la revendication 1.

**8.** Composition pharmaceutique selon la revendication 1 comprenant un composé de formule (VIII) :

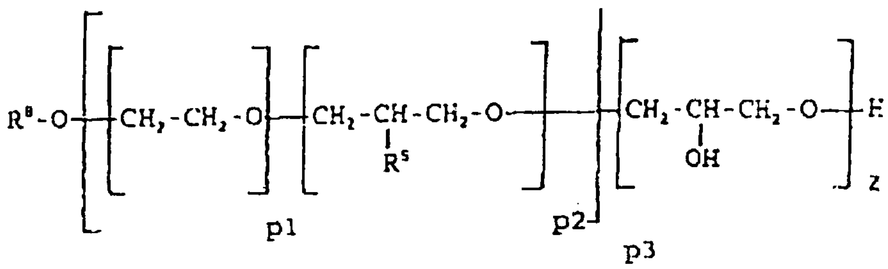

dans laquelle
$R^8$, $R^5$ et z sont définis comme dans la revendication 1 et
p1 est un entier de 0 à 20,
p2 est un entier de 0 à 20 et
p3 est un entier de 1 à 10,
à condition que p1 + p2 ≥1 et à condition que si p1 = 0 au moins un $R^5$ = H.

**9.** Composition pharmaceutique selon la revendication 1 comprenant un composé de formule (IX) :

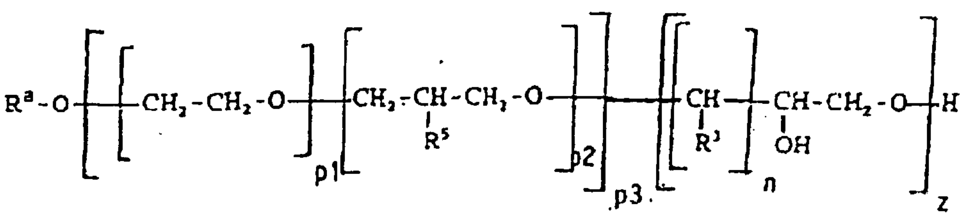

dans laquelle
$R^3$, $R^5$, $R^8$ et z sont définis comme dans la revendication 1 et
p1 est un entier de 0 à 20,
p2 est un entier de 0 à 20,
p3 est un entier de 1 à 10 et
n est un entier ≥2.

**10.** Composition pharmaceutique selon l'une quelconque des revendications précédentes, dans laquelle :

a) $R^1$ et $R^2$ sont indépendamment un groupe acyle, alcynyle, alcényle, alkyle en $C_1$ à $C_{22}$, à condition que un parmi $R^1$ ou $R^2$ soit -H ;

b) chaque $R^6$ est -H ou un groupe acyle, alcynyle, alcényle ou alkyle en $C_1$ à $C_{22}$ et est indépendamment choisi; et
c) p est un entier de 1 à 6.

**11.** Composition pharmaceutique selon l'une quelconque des revendications précédentes, dans laquelle chaque $R^6$ est -H.

**12.** Composition pharmaceutique selon l'une quelconque des revendications précédentes, dans laquelle y est 1.

**13.** Composition pharmaceutique selon l'une quelconque des revendications précédentes, dans laquelle $R^1$ est un alkyle en $C_4$ à $C_{12}$ et $R^2$ est -H.

**14.** Composition pharmaceutique selon l'une quelconque des revendications précédentes, dans laquelle p est 2 ou 3.

**15.** Composition pharmaceutique selon la revendication 1, dans laquelle le composé
est 3-(3-hexyloxy-2-hydroxy-propoxy)-propane-1,2-diol
ou
3-[2-hydroxy-3-(2-hydroxy-2-octyloxy-propoxy)-propoxy]-propane-1,2-diol.

**16.** Composition pharmaceutique selon l'une quelconque des revendications précédentes comprenant en outre un agent pharmaceutiquement actif.

**17.** Composition pharmaceutique selon l'une quelconque des revendications précédentes comprenant en outre des additifs et/ou des diluants pharmaceutiques habituels.

**18.** Composition pharmaceutique selon la revendication 16 ou 17, dans laquelle l'agent pharmaceutiquement actif est un médicament destiné à traiter des troubles de l'encéphale.

**19.** Composition pharmaceutique selon la revendication 16 ou 17, dans laquelle l'agent pharmaceutiquement actif est un médicament destiné à traiter des troubles du tractus gastro-intestinal.

**20.** Composition pharmaceutique selon la revendication 16 ou 17, dans laquelle l'agent pharmaceutiquement actif est un médicament destiné à traiter des troubles de la peau.

**21.** Composition pharmaceutique selon la revendication 16 ou 17, dans laquelle l'agent pharmaceutiquement actif est un médicament destiné à traiter un trouble respiratoire.

**22.** Utilisation d'un composé représenté par la formule (I) :

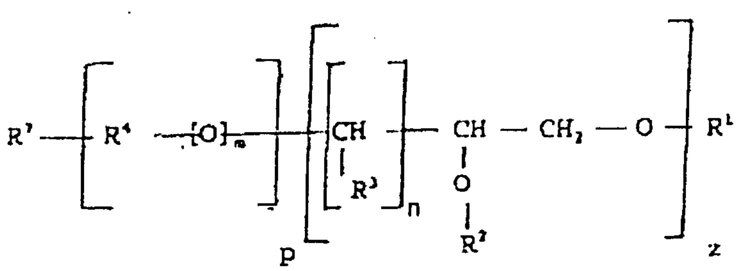

dans laquelle
$R^1$, $R^2$, $R^6$, $R^8$ et $R^9$ indépendamment à chaque occurrence représentent un hydrogène ou un groupe acyle ou hydrocarbure substitué ou non substitué, saturé ou insaturé, ramifié ou linéaire, à condition qu'au moins un parmi $R^1$ et $R^2$ soit H,
$R^3$ indépendamment à chaque occurrence représente H, OH ou -O-$R^9$,
$R^4$ indépendamment à chaque occurrence représente $-(CH_2)_x-$ ou $-CH_2-[CH(R^5)-]_yCH_2-$,
$R^5$ indépendamment à chaque occurrence représente H, OH, $R^6$ ou -O-$R^6$,
$R^7$ représente H, OH, $CH_3$ ou -O-$R^8$,
n est un entier de 0 à 6,

m est 0 ou 1,
p est un entier de 1 à 9,
x est un entier de 0 à 12,
y est un entier de 1 à 4 et
z est un entier de 1 à 8
pour la fabrication d'une composition pharmaceutique destinée à délivrer un agent pharmaceutiquement actif à l'encéphale.

**23.** Utilisation selon la revendication 22, dans laquelle la composition pharmaceutique est administrée par voie intraveineuse ou un intraartériel.

**24.** Utilisation d'un composé représenté par la formule (I) :

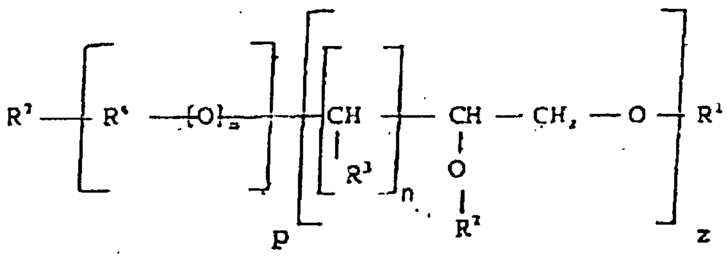

dans laquelle
$R^1$, $R^2$, $R^6$, $R^8$ et $R^9$ indépendamment à chaque occurrence représentent un hydrogène ou un groupe acyle ou hydrocarboné substitué ou non substitué, saturé ou insaturé, ramifié ou linéaire, à condition qu'au moins un parmi $R^1$ et $R^2$ soit H,
$R^3$ indépendamment à chaque occurrence représente H, OH ou $-O-R^9$,
$R^4$ indépendamment à chaque occurrence représente $-(CH_2)_x-$ ou $-CH_2-[CH(R^5)-]_yCH_2-$,
$R^5$ indépendamment à chaque occurrence représente H, OH, $R^6$ ou $-O-R^6$,
$R^7$ représente H, OH, $CH_3$ ou $-O-R^8$,
n est un entier de 0 à 6,
m est 0 ou 1,
p est un entier de 1 à 9,
x est un entier de 0 à 12,
y est un entier de 1 à 4 et
z est un entier de 1 à 8
pour la fabrication d'une composition pharmaceutique destinée à délivrer un agent pharmaceutiquement actif au tractus gastro-intestinal.

**25.** Utilisation selon la revendication 24, dans laquelle la composition pharmaceutique est encapsulée et administrée par voie orale.

**26.** Utilisation d'un composé représenté par la formule (I) :

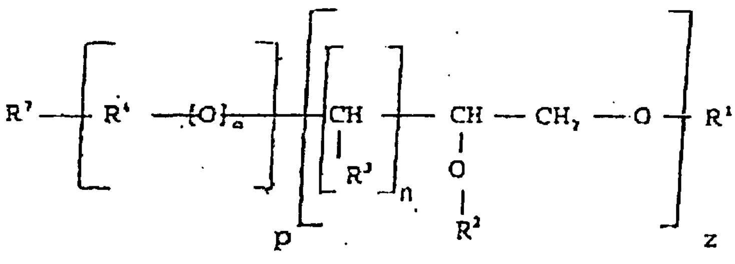

dans laquelle

$R^1$, $R^2$, $R^6$, $R^8$ et $R^9$ indépendamment à chaque occurrence représentent un hydrogène ou un groupe acyle ou hydrocarbure substitué ou non substitué, saturé ou insaturé, ramifié ou linéaire, à condition qu'au moins un parmi $R^1$ et $R^2$ soit H,
$R^3$ indépendamment à chaque occurrence représente H, OH ou -O-$R^9$,
$R^4$ indépendamment à chaque occurrence représente $-(CH_2)_x-$ ou $-CH_2-[CH(R^5)-]_yCH_2-$,
$R^5$ indépendamment à chaque occurrence représente H, OH, $R^6$ ou -O-$R^6$,
$R^7$ représente H, OH, $CH_3$ ou -O-$R^8$,
n est un entier de 0 à 6,
m est 0 ou 1,
p est un entier de 1 à 9,
x est un entier de 0 à 12,
y est un entier de 1 à 4 et
z est un entier de 1 à 8
pour la fabrication d'une composition pharmaceutique destinée à délivrer un agent pharmaceutiquement actif à la peau.

**27.** Utilisation d'un composé représenté par la formule (I) :

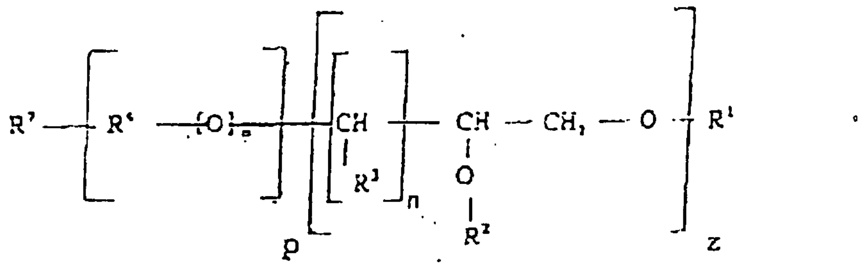

dans laquelle
$R^1$, $R^2$, $R^6$, $R^8$ et $R^9$ indépendamment à chaque occurrence représentent un hydrogène ou un groupe acyle ou hydrocarbure substitué ou non substitué, saturé ou insaturé, ramifié ou linéaire, à condition qu'au moins un parmi $R^1$ et $R^2$ soit H,
$R^3$ indépendamment à chaque occurrence représente H, OH ou -O-$R^9$,
$R^4$ indépendamment à chaque occurrence représente $-(CH_2)_x-$ ou $-CH_2-[CH(R^5)-]_yCH_2-$,
$R^5$ indépendamment à chaque occurrence représente H, OH, $R^6$ ou -O-$R^6$,
$R^7$ représente H, OH, $CH_3$ ou -O-$R^8$,
n est un entier de 0 à 6,
m est 0 ou 1,
p est un entier de 1 à 9,
x est un entier de 0 à 12,
y est un entier de 1 à 4 et
z est un entier de 1 à 8
pour la fabrication d'une composition pharmaceutique destinée à délivrer un agent pharmaceutiquement actif aux poumons.

**28.** Utilisation selon l'une quelconque des revendications 22 à 27, dans laquelle la composition pharmaceutique comprend un composé de formule (II) :

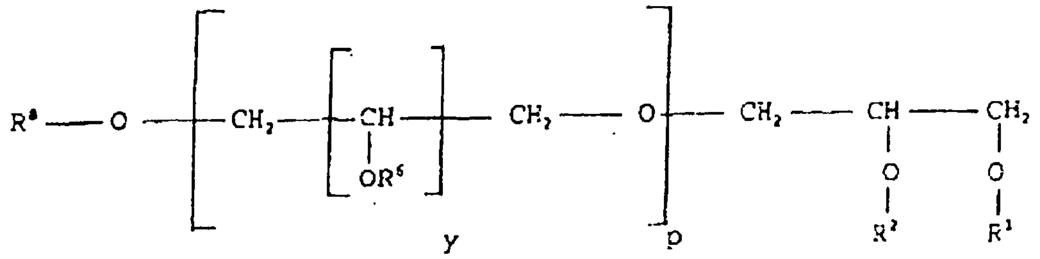

dans laquelle
$R^1$ et $R^2$ sont indépendamment H ou un groupe aliphatique substitué ou non substitué ou un groupe acyle substitué, à condition qu'au moins un parmi $R^1$ et $R^2$ soit H, chaque $R^6$ est -H ou un groupe aliphatique substitué ou non substitué ou un groupe acyle substitué ou non substitué et est indépendamment choisi ;
y est un entier de 1 à 4 ; et
p est un entier de 1 à 9.

**29.** Utilisation selon la revendication 28, dans laquelle :

a) $R^1$ et $R^2$ sont indépendamment un groupe acyle, alcynyle, alcényle ou alkyle en $C_1$ à $C_{22}$, à condition que un parmi $R^1$ ou $R^2$ soit -H ;
b) chaque $R^6$ est -H ou un groupe acyle, alcynyle, alcényle ou alkyle en $C_1$ à $C_{22}$ et est indépendamment choisi ; et
c) p est un entier de 1 à 6.

**30.** Utilisation selon la revendication 28 ou 29, dans laquelle y est 1 et chaque $R^6$ est -H.

**31.** Utilisation selon l'une quelconque des revendications 28 à 30, dans laquelle p est 2 ou 3.

**32.** Utilisation selon l'une quelconque des revendications 28 à 31, dans laquelle le composé est 3-(3-hexyloxy-2-hydroxy-propoxy)-propane-1,2-diol ou
3-[2-hydroxy-3-(2-hydroxy-2-octyloxy-propoxy)-propoxy]-propane-1,2-diol.

**33.** Utilisation selon la revendication 27, dans laquelle la composition est destinée à une administration par des moyens pulmonaires.

**34.** Utilisation d'un composé de formule générale (I) définie dans la revendication 1 pour la fabrication d'une composition pharmaceutique destinée à améliorer la pénétration des médicaments dans un tissu.

**35.** Utilisation selon la revendication 34 pour la fabrication d'une composition pharmaceutique destinée à améliorer le transport de substances actives à travers la barrière hématoencéphalique.

**36.** Utilisation selon la revendication 34 pour la fabrication d'une composition pharmaceutique destinée à améliorer la résorption de substances actives dans le tractus gastro-intestinal.

**37.** Utilisation selon la revendication 34 pour la fabrication d'une composition pharmaceutique destinée à améliorer la pénétration de substances actives dans la peau.

**38.** Utilisation d'un composé de formule générale définie dans la revendication 1 comme médiateur de solubilité pour substances actives dans des préparations pharmaceutiques.

**39.** Utilisation d'un composé de formule générale définie dans la revendication 1 comme additif pour produire des formulations de liposomes.

**40.** Utilisation selon la revendication 39, dans laquelle les formulations de liposomes sont utilisées comme préparations pharmaceutiques.

Exemplar 1

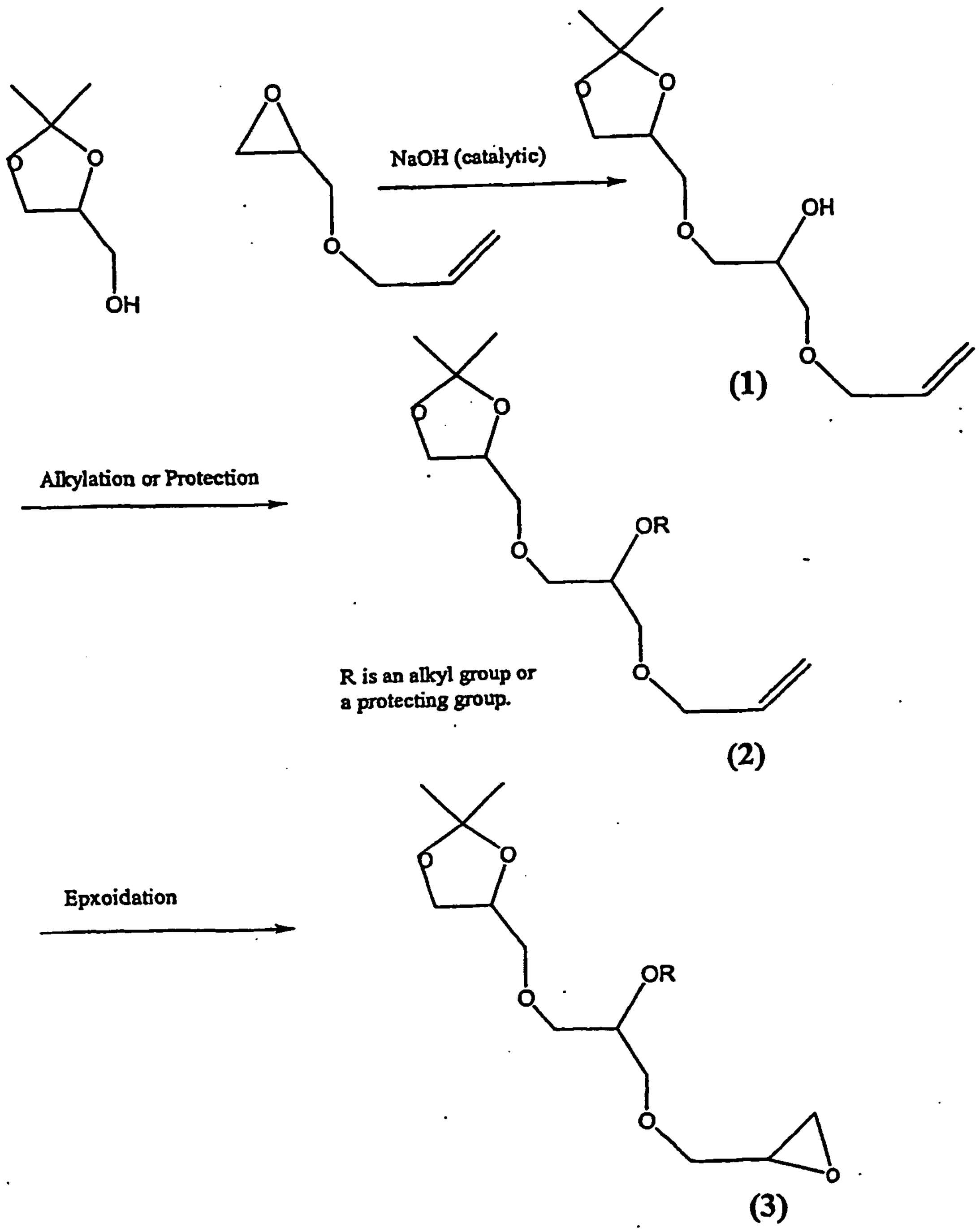


**Figure 1A**

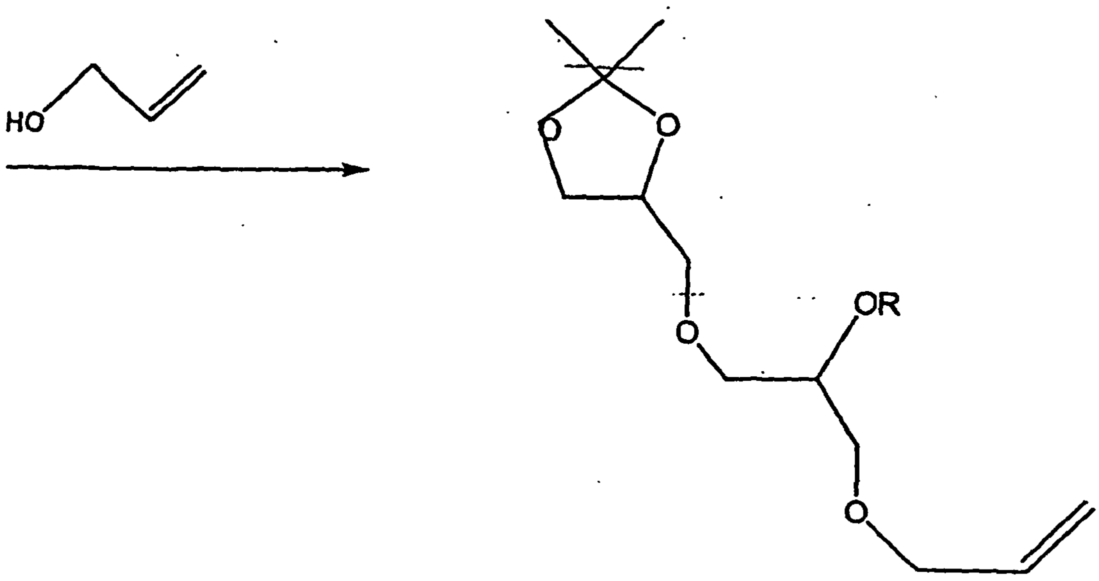

(4)

**Figure 1B**